# EUROPEAN PATENT APPLICATION

(11) **EP 4 209 272 A1**
(43) Date of publication of application: **12.07.2023**
(21) Application number: 22209210.8
(22) Date of filing: 23.11.2022
(51) Int. Cl.: B01L 3/00, G01N 1/28

(54) **TEST DEVICE FOR NUCLEIC ACID**

(30) Priority: 11.01.2022 CN 202210030057; 09.09.2022 CN 202222410660 U; 09.09.2022 CN 202222412830 U; 09.09.2022 CN 202211107998; 09.09.2022 CN 202211107997
(71) Applicant: Hangzhou Xunling Biotech Co., Ltd, Hangzhou, Zhejiand (CN)
(72) Inventor: JIN, Xia, Hangzhou (CN); XIAO, Jie, Hangzhou (CN); HU, Bin, Hangzhou (CN); LIU, Jie, Hangzhou (CN)
(74) Representative: Piotrowicz, Pawel Jan Andrzej

(57) **Abstract**

The present invention provides a test device for nucleic acid, including a sample treatment chamber, a sample reaction chamber and a test chamber which are disposed from top to bottom successively. The functions of sampling, nucleic acid purification, isothermal amplification and testing result reading by immunochromatography are integrated onto a device to prepare a simple and cute test device for nucleic acid; the device can achieve nucleic acid testing only by two rotations and can ensure that each sampling or reagent adding can reach the target area in a free falling way without any extra drainage facility. The present invention has lower costs, more convenient operation, high detection sensitivity, short time required in nucleic acid testing, and can be used for nucleic acid testing of various samples, such as human and animals.

## Description

### CROSS-REFERENCE TO RELATED APPLICATIONS

The present application claims priority to a Chinese prior application No. 2022100300570 and filed on January 11, 2022; and the present application also claims priority to Chinese prior applications No. 202222410660X and filed on September 9, 2022; No. 2022224128308 and filed on September 9, 2022; No. 2022111079986 and filed on September 9, 2022; and No. 2022111079971 and filed on September 9, 2022 by itself; all the contents of which are incorporated herein as a portion of the present invention.

### TECHNICAL FIELD

The present invention falls within the technical field of bioassay, and relates to a test device for nucleic acid, and in particular to an integrated device for isothermal amplification and testing of nucleic acid.

### BACKGROUND OF THE INVENTION

With the development of science and technology, testing technologies and equipment in the fields such as, pathogens, environmental microorganisms, biochemical indexes, tumor and organ markers and drugs have achieved great development. For example, the PCR amplifier and isothermal amplifier to which the principle of nucleic acid amplification is applied is gradually substituting the conventional smear or microscopic examination methods in the field of pathogen or environmental microorganism detection. Automatic chemiluminescent platform provides sensitive, rapid and high-throughput solutions for the test items such as, biochemical indexes and tumor markers.

The expansion of test demands and abundance of test scenes have caused point-of-care testing (POCT) to be one of the major development directions in the detection field in recent years. POCT refers to a form of testing which performs instant analysis on the sampling site to obtain a test result quickly. For example, the POCT nucleic acid testing device achieves the integration of nucleic acid extraction, amplification, fluorescence detection and all the other functions onto a small-sized instrument, which is capable of performing rapid and accurate nucleic acid testing under non-laboratory conditions.

Compared with the conventional large-scale equipment or standardized laboratory, the POCT device provides on-site test means for extensive grassroots units, remote areas or other sites without adequate medical conditions. Meanwhile, the POCT device provides a convenient and quick solution for test items with more sensitive testing time. Lots of POCT products, for example, small-sized glucometer and test paper for urine glucose have put into household scenes to complete the testing by the user in a form of self-detection.

Under lots of circumstances, people desire to complete testing in a self-detection way; moreover, in case of some field environment or being lack of professionals or equipment, people are in sore need of a quick and simple test device with low costs and accurate results and without professional training. The device should have extensive scene adaptability and safety in use.

However, there are still many problems in the existing small-sized test device: 1, the existing small-sized test device is still too expensive for ordinary people; 2, because nucleic acid testing always requires nucleic acid amplification or acquisition and analysis of fluorescence signals, and thus demands for more complex equipment and professional skills; but at present, the household self-detection equipment in China still cannot achieve nucleic acid testing; 3, even though the existing test paper self-detection products satisfy certain requirements, there are still many problems such as, inaccurate sampling and difficulties in quantitative and multiple detection; 4, the existing self-testing device has low detection sensitivity; 5, the existing self-testing device takes longer time; and 6, the existing household test device is lack of flexibility and has problems of repeated development and waste of resources.

CN113512490A provides a self-driving microfluidic detection device. The device achieves the sample addition, amplification and detection by a microfluidic diversion component. However, the microfluidic diversion process is complex and prone to be affected by environment temperature and humidity, leading to detection errors easily. WO2022/043697A1 provides a device for the analysis of biological samples; but the device needs to selectively open different regions by means of a pull rod, a piston and other control means, thus achieving the transferring of samples from one region to another region. Therefore, the device is tedious in operating process, prone to error and complex in structure.

Therefore, there is a need for a quick and simple disposable test device which has low costs, simple structure, high detection sensitivity, short testing time, safe and reliable and capable of achieving the accurate control of sample addition and testing process without no professional training.

### SUMMARY OF THE INVENTION

To make up the shortcomings in the prior art, the present invention provides a test device for nucleic acid. The functions of sampling, nucleic acid purification, isothermal amplification and testing result reading by immunochromatography are integrated onto a device; nucleic acid is absorbed by a nucleic acid adsorption membrane first, and the excessive sample flows into a filter paper storage slot; and amplification and detection of the nucleic acid are completed in a way of two rotations. The first rotation causes the nucleic acid on the nucleic acid adsorption membrane to be transferred into an amplification chamber; and the second rotation causes the amplification slot together with the amplified product to be transferred onto a test strip for detection. The test device for nucleic acid prepared in the present invention is simple and small and can ensure that each sampling or reagent adding can reach the target area in a free falling way without any extra drainage facility in the way of two rotations. Moreover, the present invention has lower costs, more convenient operation, high detection sensitivity, short time required in nucleic acid testing, and can be used for nucleic acid testing of various samples, such as human and animals.

Based on this, a first aspect of the present invention provides a test device, including a chamber used for treating a sample; a chamber used for sample reaction, where a reaction product of the sample is obtained in the chamber; and a test chamber used for detecting the reaction product; where the sample treatment chamber has a first position, a second position and a third position; the sample reaction chamber has a first position and a second position; when the sample treatment chamber and the sample reaction chamber are located in the first position, the sample treatment chamber, the sample reaction chamber and the test chamber are not in fluidic communication with each other.

In some embodiments, when the sample treatment chamber is located in the second position, the sample treatment chamber is in fluidic communication with the sample reaction chamber, and the sample reaction chamber is not in fluidic communication with the test chamber.

In some embodiments, when the sample treatment chamber is located in the third position, the sample reaction chamber is in fluidic communication with the test chamber.

In some embodiments, when the sample treatment chamber moves to the second position from the first position, the sample reaction chamber is located in the first position; when the sample treatment chamber moves to the third position from the second position, the sample reaction chamber is located in the second position, or the sample reaction chamber moves to the second position from the first position.

In some embodiments, when the sample reaction chamber is located in the second position, the sample reaction chamber is in fluidic communication with the test chamber.

In some embodiments, the sample reaction chamber moves to the second position from the first position while the sample treatment chamber moves to the third position from the second position; or the sample treatment chamber and the sample reaction chamber move simultaneously, thus driving the sample treatment chamber to move to the third position from the second position and driving the sample reaction chamber to move to the second position from the first position.

In some embodiments, the sample treatment chamber, the sample reaction chamber and the test chamber are disposed from top to bottom successively.

In some embodiments, transformation of the sample treatment chamber and the sample reaction chamber is achieved by rotation in different positions.

In some embodiments, the rotation is performed for two times; the first rotation causes the sample treatment chamber to move to the second position from the first position; and the sample reaction chamber and the test chamber keep still; at the end of the first rotation, the sample treatment chamber is in fluidic communication with the sample reaction chamber, and the sample reaction chamber is not in fluidic communication with the test chamber.

In some embodiments, the first rotation is the separate rotation of the sample treatment chamber; the sample reaction chamber and the test chamber keep still.

In some embodiments, the second rotation causes the sample treatment chamber to move to the third position from the second position; and meanwhile, the sample reaction chamber moves to the second position from the first position; at the end of the second rotation, the sample treatment chamber, the sample reaction chamber and the test chamber are in fluidic communication with each other.

In some embodiments, the second rotation is the joint rotation of the sample treatment chamber and the sample reaction chamber, while the test chamber keeps till.

In some embodiments, the sample treatment chamber is used for extracting nucleic acid substances, for example, DNA or RNA.

In some embodiments, the sample reaction chamber is used for amplifying the nucleic acid substances, thus producing an amplified product.

In some embodiments, the target substance to be tested is nucleic acid; the sample treatment chamber is used for adsorbing and purifying the nucleic acid in the sample; the sample reaction chamber is used for providing a nucleic acid amplification reagent, thus performing nucleic acid amplification.

In some embodiments, the test chamber is used for detecting the number of the amplified product or detecting the presence or absence of the amplified product.

In some embodiments, the sample reaction chamber includes a reagent for nucleic acid amplification, and the reagent exists in a dry state.

In some embodiments, the test chamber includes a transverse flow test strip, and the transverse flow test strip is used for detecting the number of the amplified product or detecting the presence or absence of the amplified product.

In some embodiments, the present invention provides a test device for nucleic acid; the sample treatment chamber is used for adsorbing the nucleic acid in the sample; the sample reaction chamber is used for completing the nucleic acid amplification; and the test chamber is used for detecting the nucleic acid in the amplified product.

In some embodiments, at the end of the first rotation, the sample treatment chamber is communicated with the sample reaction chamber; the extracted nucleic acid is transferred to the sample reaction chamber for nucleic acid amplification; at the end of the second rotation, the sample reaction chamber is communicated with the test chamber such that the amplified product is transferred onto the transverse flow test strip in the test chamber for detection.

In some embodiments, the sample treatment chamber is provided with a sample inlet; a sampling channel is connected below the sample inlet; a nucleic acid adsorption membrane is fixed at the bottom of the sampling channel, and the nucleic acid adsorption membrane is used for adsorbing nucleic acid substances in the sample.

In some embodiments, the sample reaction chamber is provided with a reaction chamber and a filter paper storage slot; the filter paper storage slot is provided with filter paper used for adsorbing the excessive sample; the reaction chamber is provided with a nucleic acid amplification membrane or fixed with a nucleic acid amplification drying agent; the test chamber is provided with a sampling hole, and the reaction product flows onto the transverse flow test strip from the sampling hole to initiate testing.

Further, when the sample treatment chamber, the sample reaction chamber and the test chamber are not communicated, the sample inlet of the sample treatment chamber is vertically communicated with the filter paper storage slot; when the sample treatment chamber is communicated with the sample reaction chamber, the sample inlet of the sample treatment chamber is vertically communicated with the reaction chamber of the sample reaction chamber; when the sample treatment chamber, the sample reaction chamber and the test chamber are communicated, the sample inlet of the sample treatment chamber, the reaction chamber of the sample reaction chamber and the sampling hole of the test chamber are communicated vertically.

The present invention achieves the amplification and detection of nucleic acid in the way of two rotations; the sample treatment chamber adsorbs nucleic acid via a nucleic acid adsorption membrane first. The first rotation causes the nucleic acid adsorption membrane adsorbed with nucleic acid to be transferred above the amplification chamber, and then nucleic acid on the nucleic acid adsorption membrane is washed into the reaction chamber with an eluant in a free falling way at the shortest distance, thus performing isothermal amplification. The second rotation causes the reaction chamber together with the amplified product to be transferred onto the transverse flow test strip; and the amplified product drips onto the test strip for detection in a free falling way. Therefore, the present invention is more convenient and flexible without errors in use.

Therefore, the present invention ensures that each sampling or reagent adding can reach the target area in a free falling way by designing two rotations. Moreover, the present invention has a compact and small structure without any extra drainage facility, which is capable of improving efficiency and detection sensitivity.

In some embodiments, both the sample treatment chamber and the sample reaction chamber have a cylindrical shape, convenient for holding and rotation.

In some embodiments, the sample treatment chamber includes an top cover and a bottom cover; the top cover is provided with a sample inlet; a sampling channel is connected below the sample inlet; a nucleic acid adsorption membrane is fixed at the bottom of the sampling channel, and the nucleic acid adsorption membrane is used for adsorbing nucleic acid in the sample; the bottom cover includes an outer wall and a bottom surface; the bottom surface is provided with a through hole; the top cover is fixed with the bottom cover, and the through hole is always aligned at the sampling channel.

In some embodiments, the sample inlet has a diameter of 5-10 mm, and the sample inlet has a cylindrical channel, and the bottom surface has a diameter the same as the sample inlet, and the height is 15-25 mm, extending downwards directly from the sample inlet; the sample reaches to the nucleic acid adsorption membrane from the sample inlet via the sampling channel.

The sample enters from the sample inlet and is capable of impacting the nucleic acid adsorption membrane with greater force after being accelerated by a certain height of sampling channel, which promotes the nucleic acid adsorption membrane to adsorb the nucleic acid in the sample more fully, thereby improving the detection sensitivity of nucleic acid.

In some embodiments, the nucleic acid adsorption membrane is a circular film and may be selected from a silicon dioxide film or polysiloxane (silica) film; and the diameter thereof is consistent with or slightly greater than the diameter of the sample inlet. The nucleic acid adsorption membrane is fixed at the bottom of the sampling channel and is required to fully cover the outlet at the bottom of the sampling channel; the whole sample must flow through the nucleic acid adsorption membrane after entering from the sample inlet, and then almost all the nucleic acid in the sample is absorbed by the nucleic acid adsorption membrane.

In some embodiments, a bulge is disposed on the periphery of the side wall of the top cover; the bottom cover is provided with a groove in fit with the bulge on the side wall; the top cover and the bottom cover are respectively fixed via the bulge and the groove, that is, the top cover and the bottom cover have fixed positions; the through hole on the bottom surface of the bottom cover is always aligned at the sampling channel; the top cover and the bottom cover are only rotated together instead of separate rotation.

In some embodiments, the side wall of the top cover has a diameter slightly smaller than that of the bottom cover such that the top cover may be sleeved into the side wall of the bottom cover; a bulge loop is disposed on the side wall of the bottom cover such that the top cover upper plane is just parallel to the highest section of the side wall of the bottom cover after the top cover is combined with the bottom cover.

In the initial position, that is, before not rotation, the sampling channel on the top cover of the sample treatment chamber and the through hole of the bottom cover are located above the filter paper storage slot; the sampling channel, the through hole and the filter paper storage slot are vertically distributed in a straight line. Therefore, nucleic acid in the sample is adsorbed by the nucleic acid adsorption membrane at the beginning of sampling; the remaining liquid flows into the filter paper storage slot after permeating the nucleic acid adsorption membrane, thus being adsorbed by the filter paper in the slot.

The reaction chamber is used for nucleic acid amplification. To make the testing process more convenient and flexible, it needs to put a dry immobilized nucleic acid amplification reagent in the reaction chamber in advance, thus avoiding repeated addition of multiple reagents from the sample inlet during the detection and preventing the reagent from being adsorbed by the nucleic acid adsorption membrane at the sample inlet to affect the isothermal amplification, leading to detection errors.

Further, a first rotary buckle is disposed on the outer wall of the sample treatment chamber, and a hollow first rotary slot is disposed on the side wall of the sample reaction chamber; in case of the first rotation, the first rotary buckle moves from one end of the first rotary slot to another end of the first rotary slot; the number of the first rotary buckle and the number of the first rotary slot are more than one.

In some embodiments, the sample treatment chamber has a narrowed diameter at the lower end of the side wall of the bottom cover such that the side wall of the bottom cover may be sleeved into the side wall of the sample reaction chamber. Therefore, after being combined with each other, the sample treatment chamber and the sample reaction chamber form a regular cylinder, which is more beautiful and also more convenient to rotation.

In some embodiments, more than one bulging first rotary buckles, for example two first rotary buckles, are disposed on the periphery of the lower end of the side wall of the bottom cover in the sample treatment chamber, and are symmetrically distributed at the lower end of the outer wall; more than one first rotary slots, for example two first rotary slots, are disposed at the top of the outer wall of the sample reaction chamber, and are symmetrically distributed at the top of the outer wall; the first rotary slot is a strip-type transverse hollow groove disposed on the outer wall; when the lower end of the side wall of the bottom cover is sleeved into the side wall of the sample reaction chamber, the bulging first rotary buckle is just located in the hollow first rotary slot.

In some embodiments, both left end and right end of the first rotary slot are respectively provided with bulged limiting blocks. In the initial position, the first rotary buckle is located at the leftmost end of the first rotary slot, blocked and fixed by the limiting block; at the end of nucleic acid extraction (after sampling and the nucleic acid in the sample is absorbed by the nucleic acid adsorption membrane), first rotation is performed; the first rotary buckle needs to overcome the blocking of the left-end limiting block to slide rightwards along with the first rotary slot until the first rotary buckle overcomes the right-end limiting block to enter into the rightmostend and be blocked and fixed by the limiting block and may not continue to rotate rightwards; at this time, the sampling channel and the nucleic acid adsorption membrane are just transferred above the amplification chamber and ready for the nucleic acid washing and isothermal amplification.

The first rotation is to fix the test device for nucleic acid with one hand and to hold the upper end of the test device for nucleic acid with another hand for rotation, which is a kind of rotation of the sample treatment chamber relative to the sample reaction chamber.

The nucleic acid adsorption membrane has a first position and a second position; the first position refers that the nucleic acid adsorption membrane is located above the filter paper storage slot; the second position refers that the nucleic acid adsorption membrane is located above the reaction chamber; when the first rotary buckle is located at one end of the first rotary slot, the nucleic acid adsorption membrane is located in the first position; when the first rotary buckle moves to another end of the first rotary slot, the nucleic acid adsorption membrane is located in the second position.

When the nucleic acid adsorption membrane is located in the first position, the test device for nucleic acid is located in the initial position, and it begins to add samples; at the end of the first rotation, the nucleic acid adsorption membrane is located in the second position, and at this time, the sampling channel, the through hole and the reaction chamber are vertically distributed in a straight line, and it may begin to add the eluant and perform the isothermal amplification of the nucleic acid.

It may be understood that the first position and the second position of the nucleic acid adsorption membrane are relative to the sample reaction chamber; in case of the second rotation, the nucleic acid adsorption membrane moves together with the sample reaction chamber. Therefore, the nucleic acid adsorption membrane has no change of position relative to the sample reaction chamber, but with respect to the test chamber, the nucleic acid adsorption membrane further has a third position because the nucleic acid adsorption membrane and the reaction chamber are together transferred above the sampling hole of the transverse flow test strip after the second rotation.

In some embodiments, the test chamber includes a test strip top cover and a test strip bottom cover; the transverse flow test strip is fixed in the test strip top cover and the test strip bottom cover, used for detecting the nucleic acid in the amplified product.

The test strip top cover is provided with a sampling hole and a test result observation window; the sampling hole is aligned at the sampling test position of the test strip; the test result observation window is aligned at a test result reading window of the test strip, used for reading the test result.

Further, a hollow second rotary slot is disposed on the side wall of the test chamber; a second rotary buckle is disposed on an outer wall of the sample reaction chamber; in case of the second rotation, the second rotary buckle moves from one end of the second rotary slot to another end of the second rotary slot; the number of the second rotary buckle and the number of the second rotary slot is more than one.

In some embodiments, a cylindrical groove is disposed in the test strip top cover; and a second rotary slot is disposed on the groove wall; a second rotary buckle is disposed at the lower portion of the outer wall of the sample reaction chamber; when the sample reaction chamber rotates relative to the test chamber, the second rotary buckle moves from one end of the second rotary slot to another end of the second rotary slot.

In some embodiments, the sample reaction chamber has a narrowed lower diameter on the outer wall such that the lower portion of the outer wall of the sample reaction chamber may be sleeved into the cylindrical groove of the test strip top cover. In this way, after the sample reaction chamber is combined with the test chamber, the sample treatment chamber, the sample reaction chamber and the cylindrical groove jointly form a regular cylinder.

In some embodiments, more than one bulging second rotary buckles, for example two second rotary buckles, are disposed at the lower portion of the outer wall of the sample reaction chamber, and are symmetrically distributed at the lower end of the outer wall; more than one second rotary slots, for example two second rotary slots, are disposed at the upper portion of the wall of the cylindrical groove, and are symmetrically distributed on the groove wall; the second rotary slot is a strip-type transverse hollow groove disposed on the groove wall; when the lower portion of the outer wall of the sample reaction chamber is sleeved into the cylindrical groove, the bulging second rotary buckle is just located in the hollow second rotary slot.

In some embodiments, both left end and right end of the second rotary slot are respectively provided with bulged limiting blocks. In the process of first rotation, the second rotary buckle and the second rotary slot always keep still in position; the second rotary buckle is located at the leftmost end of the second rotary slot, blocked and fixed by the left-end limiting block; at the end of nucleic acid amplification, second rotation is performed; the second rotary buckle needs to overcome the blocking of the left-end limiting block to slide rightwards along with the second rotary slot until the second rotary buckle overcomes the right-end limiting block to enter into the rightmost end and be blocked and fixed by the limiting block; at this time, the nucleic acid adsorption membrane and the reaction chamber are jointly transferred above the sampling hole of the test strip.

The second rotation is to fix the test device for nucleic acid with one hand and to hold the middle or upper portion of the test device for nucleic acid with another hand for rotation, which is a kind of rotation of the sample reaction chamber relative to the test chamber.

In some embodiments, when the sample reaction chamber rotates relative to the test chamber, the sample treatment chamber rotates with the sample reaction chamber, and the nucleic acid adsorption membrane is always located above the reaction chamber.

Further, the number of the first rotary buckle, the number of the first rotary slot, the number of the second rotary buckle and the number of the second rotary slot are two, and these are distributed symmetrically.

Further, the reaction chamber is a cylindrical chamber and cut through from top to bottom; a gasket is disposed at the periphery of the lower end of the cylindrical chamber; when the reaction chamber slides on the bottom surface of the cylindrical groove, liquid in the reaction chamber is free of spilling to the reaction chamber under the protection of the gasket; the bottom surface of the cylindrical groove is provided with a sampling hole; when the reaction chamber rotates above the sampling hole, the amplified product drips onto the test strip from the sampling hole in a free falling way for nucleic acid testing.

The reaction chamber is a cut-through cylinder; there is neither an upper bottom surface nor a lower bottom surface. No upper bottom surface is convenient to directly wash the sample into the reaction chamber from the upper nucleic acid adsorption membrane; and no lower bottom surface is convenient to transfer the amplified product in the reaction chamber onto the sampling hole at the bottom board inside the test device for nucleic acid; at this time, liquid in the reaction chamber may smoothly drip onto the test strip for nucleic acid due to being lack of the support from the bottom board.

The reaction chamber has no lower bottom surface, but is disposed on the test strip bottom cover; a gasket is disposed on the periphery of the lower end of the reaction chamber such that liquid in the reaction chamber will not leak away from the gap of the side wall of the reaction chamber and from the surface of the test strip bottom cover; moreover, when the second rotation is performed after completing the amplification, the reaction chamber will slide on the surface of the test strip bottom cover; with the protection of the gasket, the sliding may not cause the reaction liquid to leak away from the reaction chamber.

In some embodiments, a gasket groove is disposed on the periphery of the lower end of the reaction chamber, used for fixing the position of the gasket.

Further, a layer of drainage groove is disposed on the inner wall of the reaction chamber; the drainage groove is composed of multiple trapezoid columns spaced equally which are disposed on the inner wall; a drainage channel is formed between the adjacent two trapezoid columns.

Since there is a trace amount of sample for nucleic acid amplification, how to ensure the drainage of the full sample into the reaction chamber is a very crucial step to guarantee the detection sensitivity of nucleic acid. Drainage grooves are disposed around the inner wall of the reaction chamber, which may drain the sample to be tested into the reaction chamber to the maximum extent, thus avoiding leakage.

In some embodiments, the trapezoid columns are arranged directly from the upper end of the reaction chamber to the lower end of the reaction chamber.

The trapezoid columns are arranged directly from the upper end of the reaction chamber to the lower end of the reaction chamber such that the sample may be drained to the lower end from the upper end of the reaction chamber all the time. This is mainly because an immobilization membrane or a drying agent for nucleic acid amplification is placed at the lower end of the reaction chamber; only when the sample is drained to the lower end from the upper end of the reaction chamber, the sample may be ensured to fully contact with the immobilization membrane or drying agent, thus improving the amplification efficiency.

In some embodiments, an upward-narrowing step is disposed on the upper end of each trapezoid column; the drainage channel has a width of 0.1-0.5 mm.

The upward-narrowing step at the upper end of the trapezoid column may contact with the upper nucleic acid adsorption membrane, thus assisting in absorbing the sample from the upper part into the drainage channel, thus achieving full drainage.

In some embodiments, the upward-narrowing step has a height of 0.2 mm.

The drainage channel formed between each trapezoid column has a consistent width; the narrower the width of the drainage channel is, the better the drainage effect is. However, too narrow drainage channel will increase the difficulty of the manufacturing process. Therefore, it needs to choose a proper width of the drainage channel.

In some embodiments, the number of the trapezoid columns is 13, and there are 13 drainage channels arranged evenly along with the inner wall of the reaction chamber.

In some embodiments, the drainage channel has a width of 0.5 mm.

In some embodiments, each of the trapezoid column has a bottom width of 0.9 mm; the distance protruding from the inner wall of the reaction chamber to the outside is 1.25 mm, and the height is 3.7 mm.

The configuration of width and number of the drainage channel (the number of the drainage channel determines the number and arrangement of the trapezoid column, thus determining the bottom width of each trapezoid column) determines whether the drainage effect can be improved to the maximum extent or not. The cylinder of the reaction chamber provided herein has a cross section diameter of 6 mm. A great number of studieshave shown that when the width of the drainage channel is 0.5 mm and the trapezoid column has a bottom width of 0.9 mm, and the distance protruding from the inner wall of the reaction chamber to the outside is 1.25 mm, a better drainage effect may be achieved.

Further, a heating hole is disposed at the bottom of the test chamber, and the heating hole is used to supply a heat source for the nucleic acid amplification in the sample reaction chamber.

In some embodiments, the heating hole is located on the test strip bottom cover; and the position of the heating hole is just facing towards the lower portion of the reaction chamber, used for supplying a heat source for the nucleic acid amplification.

In some embodiments, the heat source for the nucleic acid amplification may be supplied by configuring a heating hole; the heating hole is matched with a constant-temperature heating equipment; the constant-temperature heating equipment supplies a heat source for the reaction chamber via the heating hole, thus performing isothermal amplification.

In some embodiments, the heat source of the isothermal amplification of nucleic acid may be also directly disposed inside the test device for nucleic acid. In this way, it is unnecessary to further supply a constant-temperature heating equipment.

It may be understood that the reaction chamber has a first position and a second position; the first position refers that the reaction chamber is located above the heating hole; the second position refers that the reaction chamber is located above the sampling hole; when the second rotary buckle is located at one end of the second rotary slot, the reaction chamber is located in the first position; when the second rotary buckle moves to another end of the second rotary slot, the reaction chamber is located in the second position.

The reaction chamber is always located in the first position from when the test device for nucleic acid is located in the initial position until the completion of the first rotation. This is because the first rotation only refers to the rotation of the sample treatment chamber, and the sample reaction chamber keeps still.

During the second rotation, the nucleic acid adsorption membrane rotates together with the sample reaction chamber such that the reaction chamber rotates above the sampling hole from the heating hole, thus achieving sampling detection.

Further, the nucleic acid amplification membrane includes a first reaction membrane and a second reaction membrane; the first reaction membrane is laid on the lower end of the reaction chamber; the second reaction membrane and the first reaction membrane are placed horizontally, or the second reaction membrane is vertically placed above the first reaction membrane; or the second reaction membrane and the first reaction membrane are placed vertically.

In some embodiments, the first reaction membrane is an immobilization membrane of a recombinase reagent required by nucleic acid amplification; the second reaction membrane is an immobilization membrane of PEG buffer reagent required by nucleic acid amplification. Studies prove that the recombinase reagent and the PEG buffer reagent must be placed separately before the amplification, otherwise the recombinase is easily wrapped by PEG during the amplification, thus affecting the amplification efficiency. Therefore, two layers of immobilized reagent reaction membranes need to be configured.

Further, the first reaction membrane is laid on the lower end of the reaction chamber; the second reaction membrane is located at the central position of the cylinder of the reaction chamber and vertically placed above the first reaction membrane.

Experimental results show that the arrangement position of the two layers of reaction membranes also will affect the drainage effect of the sample; when the second reaction membrane is vertically placed above the first reaction membrane and located at the central position, it will assist improving the drainage effect, thus improving the amplification efficiency and improving the detection sensitivity of nucleic acid.

Further, the reaction chamber is fixed with two nucleic acid amplification drying agents, respectively a first drying agent and a second drying agent; the first drying agent and the second drying agent are spaced by a division bar disposed at the bottom surface of the reaction chamber.

The first drying agent is a recombinase reagent required by nucleic acid amplification; the second drying agent is a PEG buffer reagent required by nucleic acid amplification; the first drying agent and the second drying agent are respectively disposed at both sides of the division bar first, and dried or frozen-dried to a drying agent. During detection, an eluant is added such that the two reagents are redissolved and mixed for nucleic acid amplification. Therefore, the division bar may be not too high, resulting in mixing difficulty after being redissolved.

In another aspect, the present invention provides a test system for nucleic acid, including a test device for nucleic acid and a reagent, where the reagent includes a lysis solution, an eluant and a nucleic acid amplification reagent; the nucleic acid amplification reagent is a dry immobilized reagent, including a first immobilized reagent and a second immobilized reagent; the first immobilized reagent contains an enzyme, and the second immobilized reagent contains PEG.

The nucleic acid amplification reagent at least includes a recombinase, a primer probe combination, DNTP, ATP, dithiothreitol, PEG, Mg²⁺ and the like; PEG is used for supplying reaction environment; and Mg²⁺ is an initiating agent of the isothermal amplification.

To make the testing process more convenient and flexible, a dry immobilized nucleic acid amplification reagent is placed in the test device for nucleic acid of the present invention in advance, thus avoiding repeated addition of multiple reagents from the sample inlet during the detection and preventing the reagent from being adsorbed by the nucleic acid adsorption membrane at the sample inlet to affect the isothermal amplification, leading to detection errors.

The nucleic acid amplification reagent contains more complex components, after the steps of extraction, immobilization and drying treatment, it needs to be further considered whether the immobilized reagent can be kept in the reaction chamber for a long time and whether the immobilized reagent affects the nucleic acid amplification after being redissolved. To achieve a good amplification effect, PEG needs to be added to the nucleic acid amplification reagent; PEG molecular weight is preferably 20000-40000. In this present invention, a great number of studies have proved that PEG and other reagents, for example, recombinase, in the nucleic acid amplification reagent must be separated for drying or immobilization, or, the isothermal amplification of nucleic acid will be affected seriously, thus affecting the detection sensitivity. The reason may be as follows: PEG will be shrunk in the drying process to wrap enzymes, and is difficult to restore enzymatic activity after being redissolved in later period, which will cause the failure of the isothermal amplification of nucleic acid.

Further, the first immobilized reagent further contains a primer probe, a single-strand binding protein, a protein cofactor, and DNTP.

In some embodiments, the second immobilized reagent may further contain magnesium acetate.

The primer probe may be dried and immobilized with an enzyme, but may not dried with PEG. The reason may be that PEG may also affect the concentration of the primer probe.

Further, the test device for nucleic acid includes a sample treatment chamber, a sample reaction chamber and a test chamber which are disposed from top to bottom successively; the sample treatment chamber is used for adsorbing nucleic acid in the sample; the sample reaction chamber is used for completing the nucleic acid amplification; the test chamber is used for detecting nucleic acid in the amplified product.

Further, the sample reaction chamber includes a reaction chamber used for nucleic acid amplification; the nucleic acid amplification reagent is disposed in the reaction chamber of the sample reaction chamber in advance.

Further, the nucleic acid amplification reaction reagent is an immobilization membrane or a drying agent.

The immobilization membrane of the present invention may be made of silica gel, glass microfiber filter paper/membrane and the like; the membrane has a thickness of about 1 mm, and is circular or any other shape, preferably, a circular membrane having a diameter of 6 mm. The nucleic acid amplification reagent may be immobilized on the membrane in the way of drying, freeze-drying and the like; and the drying temperature should be not greater than 50°C.

Further, when the nucleic acid amplification reaction reagent is an immobilization membrane, the immobilization membrane includes a first reaction membrane and a second reaction membrane; the first reaction membrane contains a first immobilized reagent, and the second reaction membrane contains a second immobilized reagent; when the nucleic acid amplification reagent is a drying agent, the drying agent includes a first drying agent and a second drying agent; the first drying agent contains a first immobilized reagent, and the second drying agent contains a second immobilized reagent; and when the nucleic acid amplification reagent is a drying agent, a division bar need to be disposed at the bottom of the reaction chamber; and the first drying agent is spaced from the second drying agent by the division bar.

In some embodiments, the drying agent may be prepared by drying or freeze-drying; and the drying temperature should be not greater than 50°C.

In some embodiments, the division bar at the bottom of the reaction chamber may be in a form of partition sheet in the middle of two-flavor hot pot as long as it has the effect of separating two reagents. Two reagents may be firstly added at both sides of the division bar in the reaction chamber respectively, and then frozen-dried or dried, thus preparing a test device for nucleic acid pre-equipped with nucleic acid amplification reagents.

In some embodiments, the height of the division bar at the bottom of the reaction chamber may be not too high, being generally 2 mm as long as two reagents may be separated in advance. After being redissolved for isothermal amplification, the two reagents still need to be mixed for reaction, thus preventing the condition of difficult mixing due to too high division bar.

Further, the lysis solution includes tris(hydroxymethyl)aminomethane (Tris), ethylene diamine tetraacetic acid (EDTA), guanidinium isothiocyanate and Triton 100.

After the nucleic acid sample lysed by the lysis solution is adsorbed by the nucleic acid adsorption membrane, even though the excessive sample flows into the filter paper storage slot, there is still lysis solution residual on the nucleic acid adsorption membrane, and then the remaining lysis solution is washed into the reaction chamber by the eluant for isothermal amplification of nucleic acid. Therefore, components in the lysis solution neither affect the isothermal amplification of nucleic acid, nor affect the testing process of nucleic acid, or, the detection sensitivity of nucleic acid will be affected, resulting in leak or mistaken detection.

The lysis solution provided by the present invention may not cause adverse effect on the nucleic acid amplification. Therefore, after the nucleic acid in the sample is absorbed by the nucleic acid adsorption membrane, it is unnecessary to add a cleaning agent for washing; and the target nucleic acid may be eluted by an eluant for isothermal amplification. The lysis solution is particularly suitable for the test device for nucleic acid provided in the present invention.

Further, the sample treatment chamber includes a nucleic acid adsorption membrane, and the nucleic acid adsorption membrane is a silicon dioxide GF/C membrane or a silica membrane.

Further, the eluant includes magnesium acetate, EDTA and Tris.

After being added from the sample inlet, the eluant may elute the nucleic acid absorbed on the nucleic acid adsorption membrane into the reaction chamber for isothermal amplification.

Mg²⁺ is an initiating agent of the isothermal amplification, and may be added to the eluant for eluting nucleic acid. Mg²⁺ is added to the reaction chamber as the eluant elutes the nucleic acid on the nucleic acid adsorption membrane, which is free of affecting the elution effect of nucleic acid and may smoothly initiate the isothermal amplification of nucleic acid.

The eluant provided herein may be used to efficiently elute nucleic acid into the reaction chamber and initiate the isothermal amplification of nucleic acid, thus effectively improving the detection sensitivity of nucleic acid.

Further, the sample treatment chamber is provided with a sample inlet without a cover; the lysis solution or the eluant is placed into a reagent bottle; and the reagent bottleneck is capable of being sealed with the sample inlet.

It is unnecessary to set a cover on the sample inlet of the test device for nucleic acid provided herein because any impurity, even dust may be also blocked by the nucleic acid adsorption membrane.

After the isothermal amplification of nucleic acid, the content of nucleic acid to be tested will rise greatly and there exists a risk of biological contamination. Therefore, when a reagent bottle is used to add the eluant, the neck of the reagent bottle may be directly screwed with the sample inlet, thus playing the role of sealing and pollution prevention.

In a further aspect, the present invention provides a method for testing nucleic acid by a test system for nucleic acid, including the following steps of:
(1) adding a lysis solution to a sample to complete sample lysis;
(2) adding the lysed sample from the sample inlet;
(3) completing the first rotation;
(4) adding an eluant from the sample inlet;
(5) turning on a constant-temperature heating device for nucleic acid amplification;
(6) completing the second rotation;
(7) reading a test result from an observation window of the test strip top cover.

The test system for nucleic acid provided by the present invention is simple, convenient, integrated and the like, which reduces the demands of conventional nucleic acid testing for the laboratory, equipment and personnel skills. Therefore, the test system for nucleic acid of the present invention can be used in the field, clinics, pet stores, and the like.

In some embodiments, the test system for nucleic acid provided by the present invention can be used for nucleic acid testing of pathogens.

In some embodiments, the test system for nucleic acid provided by the present invention can be used for nucleic acid testing of pet pathogens (e.g., feline herpesvirus), pathogens of large livestock, plant pathogens or food pathogens.

Beneficial effects pf the present invention are as follows:
1. The test device for nucleic acid has an improved structure; the nucleic acid testing can be completed in the way of two rotations; moreover, the present invention can ensure that each sampling or reagent adding can reach the target area in a free falling way without any extra drainage facility, which is capable of improving efficiency and detection sensitivity.
2. A sampling channel having a certain height is configured and the sample flows from the sample inlet and is capable of impacting the nucleic acid adsorption membrane with greater force after being accelerated by a certain height of sampling channel, which promotes the nucleic acid adsorption membrane to adsorb the nucleic acid in the sample more fully, thereby improving the detection sensitivity of nucleic acid.
3. Drainage grooves are disposed in the reaction chamber; suitable width and amount of the drainage grooves are screened to improve the amplification efficiency of nucleic acid.
4. The arrangement modes of the first reaction membrane and the second reaction membrane are designed to further improve the drainage effect and improve the amplification efficiency of nucleic acid.
5. The present invention is simple and small, has low costs and convenient operation.
6. The present invention has high detection sensitivity.
7. The present invention has short testing time.
8. The present invention can be used for human, animals, and the like, and has extensive scope of application.

### BRIEF DESCRIPTION OF DRAWINGS

FIG. 1 is a structure diagram showing a test device for nucleic acid in Example 1;
FIG. 2 is a cross-section diagram of the test device for nucleic acid in Example 1;
FIG. 3 is a schematic diagram showing a disassembled structure of the test device for nucleic acid in Example 1;
FIG. 4 is a structure diagram showing a top cover of a sample treatment chamber in Example 1;
FIG. 5 is a structure diagram showing a bottom cover of the sample treatment chamber in Example 1;
FIG. 6 is a structure diagram showing the sample treatment chamber in Example 1;
FIG. 7 is a cross-section diagram showing the sample treatment chamber in Example 1;
FIG. 8 is a structure diagram showing a sample reaction chamber in Example 1;
FIG. 9 is a schematic diagram showing a disassembled structure of a test chamber in Example 1;
FIG. 10 is a structure diagram showing a transverse flow test strip in Example 1;
FIG. 11 is a structure diagram showing a reaction chamber of the sample reaction chamber in Example 1 (seen from bottom to top);
FIG. 12 is a schematic diagram showing a disassembled structure of the reaction chamber of the sample reaction chamber in Example 1;
FIG. 13 is a structure diagram showing the reaction chamber of the sample reaction chamber in Example 1 (seen from top to bottom); a first reaction membrane and a second reaction membrane are placed in the reaction chamber, and the second reaction membrane is vertically placed above the first reaction membrane;
FIG. 14 is a structure diagram showing the reaction chamber of the sample reaction chamber in Example 1 (seen from top to bottom); a division bar is disposed in the middle of the reaction chamber;
FIG. 15 is a schematic diagram showing the rotation and a change of state of the test device for nucleic acid in Example 2;
FIG. 16 is a structure diagram showing that the test device for nucleic acid in Example 3 is sealed with a reagent bottle;
FIG. 17 shows a standard colorimetric card of a test result of the transverse flow test strip in Example 4.

### DETAILED DESCRIPTION OF THE INVENTION

The structures or technical terms used in the present invention are further described in the following. Unless otherwise indicated, they are understood or interpreted according to ordinary terms and definitions in the art.

### Detection

Detection denotes assaying or testing whether a substance or material exists, for example, but not limited to, chemicals, organic compounds, inorganic compounds, metabolites, drugs or drug metabolites, organic tissues or metabolites of organic tissues, nucleic acid, proteins or polymers. Moreover, detection denotes testing the number of a substance or material. Further, assay also denotes immunoassay, chemical detection, enzyme detection and the like.

### Samples

The samples that can be detected by the detecting device or collected by the collector of the present invention include biological liquid (e.g. case liquid or clinical samples). Liquid samples or fluid specimens may be derived from solid or semi-solid samples, including excreta, biological tissues and food samples. Solid or semi-solid samples are transformed into liquid samples by any proper method, for example, mixed, mashed, macerated,incubated, dissolved into a proper solution (for example, water, phosphate solution or other buffer solutions), and solid samples are digested by zymolysis. "Biological samples" include samples from animals, plants and food, for example, including urine, saliva, blood and components thereof, spinal fluid, vaginal secretion, semen, faeces, sweat, secreta, tissues, organs, tumors, cultures of tissues and organs, cell culture and medium from human or animals. The preferred biological sample is urine, preferably, the biological sample is saliva, sputum, nasal secretion, and the like. Food samples include substances processed from food, final products, meat, cheese, wine, milk and drinking water. Plant samples are derived from any plants, plant tissues, plant cell cultures and media. "Environmental samples" are derived from the environment (for example, liquid samples, wastewater samples, soil texture samples, underground water, seawater and effluent samples from lakes and other water bodies). Environmental samples may further include sewage or other waste water.

Any analyte can be detected using the appropriate detecting element or testing element of the present invention. Preferably, the present invention is used to detect nucleic acid in blood, saliva and urines. Any form of samples above, either initially solid or liquid, can be collected by the sample treatment chamber 2 in the present invention, as long as these liquid or liquid samples can be absorbed by the nucleic acid absorbing element in the sample treatment chamber 2; and the nucleic acid absorbing element is located in the sample treatment chamber 2, and is capable of absorbing nucleic acid in the liquid sample or fluid sample, thus maintaining nucleic acid in the fluid sample into the absorbing element. The nucleic acid absorbing element can be any material capable of absorbing nucleic acid in liquid such as sponge, filter paper, polyester fiber, gel, non-woven fabric, cotton, polyester membrane, yarn, flocking, etc. In the present invention, a nucleic acid absorbing membrane 9 is preferably used as the nucleic acid absorbing element.

### Downstream and upstream

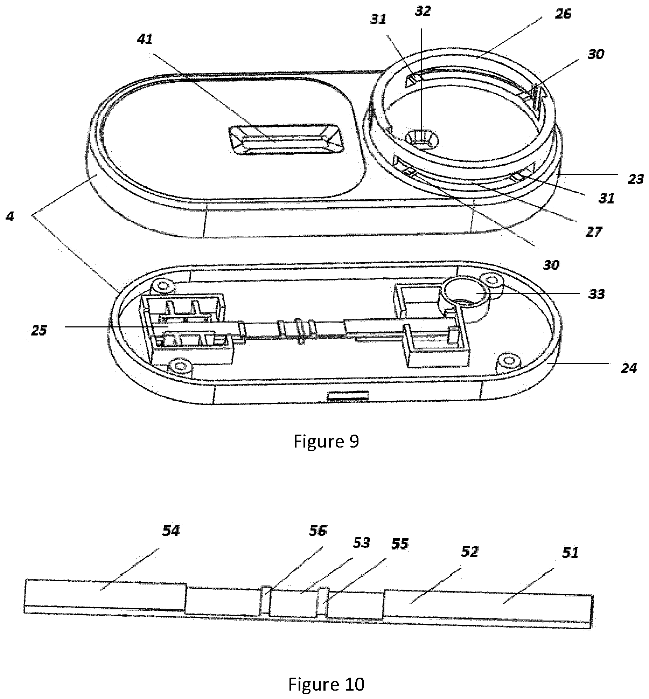

Downstream or upstream is divided according to a flow direction of a liquid, generally, a liquid or fluid flows to a downstream area from an upstream area. The downstream area receives the liquid from the upstream area, and a liquid also may flow to a downstream area along an upstream area. Here, downstream or upstream is generally divided according to a flow direction of a liquid, for example, on some materials where capillary force is utilized to promote the flow of a liquid, a liquid may overcome gravity to flow towards an opposite direction to the gravity; and at this time, downstream or upstream is divided according to a flow direction of the liquid. For example, as shown in Figure 10, the test device for nucleic acid 1 mentioned in the present invention has a sample inlet 7 for adding a sample, a sample treatment chamber 2, a sample reaction chamber 3 and a test chamber 4; the sample is added from the sample inlet 7 and flows into the sample treatment chamber 2; the sample treatment chamber 2 adsorbs nucleic acid in the sample via a nucleic acid adsorption membrane 9 first. The first rotation causes the nucleic acid adsorption membrane 9 adsorbed with nucleic acid to be transferred above an amplification chamber 15 of the amplification reaction chamber 3, and then nucleic acid on the nucleic acid adsorption membrane 9 is washed into the reaction chamber 15 with an eluant in a free falling way at the shortest distance, thus performing isothermal amplification. The sample treatment chamber 2 is located upstream of the sample reaction chamber 3, and the sample reaction chamber 3 is located at the downstream.The second rotation causes the reaction chamber 15 of the sample reaction chamber 3 together with the amplified product to be transferred onto the transverse flow test strip 25 of the test chamber 4; and the amplified product drips into the test strip 25 for detection in a free falling way. Therefore, the present invention is more convenient and flexible without errors in use. Further, the sample reaction chamber 3 is located upstream of the test chamber 4, and the test chamber 4 is located at the downstream. The present invention ensures that each sampling or reagent adding can reach the target area from the upstream to the downstream in a free falling way by designing two rotations. Moreover, the present invention has a compact and small structure without any extra drainage facility, which is capable of improving efficiency and detection sensitivity.

### Fluidic communication

Fluidic communication means that liquid can flow from one place to another place. The flow process may pass through some physical structures, to play a guiding role. The "passing through some physical structures" here means that liquid passes through the surface of these physical structures or their internal space and flows to another place passively or actively, where passivity is usually caused by external forces, such as the flow of the capillary action and air pressure action. The flow here may also be a flow due to self-action (gravity or pressure) of the liquid, and also may be a passive flow. The fluid under the action of air pressure may be a forward flow, or also a reverse flow; or a fluid is urged to flow to another position from a position under the action of air pressure. Here, the flow does not mean that a liquid is necessarily present, but indicates a relationship or state between two objects under some circumstances. In case of presence of liquid, it can flow from one object to another. Here it means the state in which two objects are connected. In contrast, if there exists no liquid flow state between two objects, and liquid exists in or above one object but cannot flow into or on another object, it is a non-flow, non-liquid flow state.

### Testing element

The "testing element" used herein refers to an element that can be used to detect whether a sample or a sample contains an interested analyte. Such testing can be based on any technical principles, such as immunology, chemistry, electricity, optics, molecular science, nucleic acids, physics, etc. The testing element can be a lateral flow test strip that can detect a variety of analytes. Of course, other suitable testing elements can also be used in the present invention.

Various testing elements can be combined for use in the present invention. One form of the testing elements is test paper or transverse-flow test paper. The test papers used for analyzing the analyte (e.g., nucleic acid, and the like) in samples can be of various forms such as immunoassay or chemical analysis. The analysis mode of non-competition law or competition law can be adopted for test papers. A test paper generally contains a water absorbent material that has a sample application area, a reagent area and a testing area. Fluid or liquid samples are added to the sample application area and flow to the reagent area through capillary action. If analyte exists in the reagent area, samples will bind to the reagent. Then, samples continue to flow to the testing area. Other reagents such as molecules that specifically bind to analyte are fixed in the testing area. These reagents react with the analyte (if any) in the sample and bind to the analyte in this area, or bind to a reagent in the reagent area. Marker used to display the detection signal exists in the reagent area or the detached labeled area.

Typical non-competition law analysis mode: if a sample contains analyte, a signal will be generated; and if not, no signal will be generated. Competition law: if no analyte exists in the sample, a signal will be generated; and if analyte exists, no signal will be generated.

The testing element can be a test paper, which can be water absorbent or non-absorbing materials. The test paper can contain several materials used for delivery of liquid samples. One material can cover the other material.For example, the filter paper covers the nitrocellulose membrane. One area of the test paper can be of one or more materials, and the other area uses one or more other different materials. The test paper can stick to a certain support or on a hard surface for improving the strength of holding the test paper.

Analyte is detected through the signal generating system. For example, one or more enzymes that specifically react with this analyte is or are used, and the above method of fixing the specifically bound substance on the test paper is used to fix the combination of one or more signal generating systems in the analyte testing area of the test paper. The substance that generates a signal can be in the sample application area, the reagent area or the testing area, or on the whole test paper, and one or more materials of the test paper can be filled with this substance. The solution containing a signifier is added onto the surface of the test paper, or one or more materials of the test paper is or are immersed in a signifier-containing solution, and the test paper containing the signifier solution is made dry.

Each area of the test paper can be arranged in the following way: sample application area, reagent area, testing area, control area, area determining whether the sample is adulterated, and liquid sample absorbing area. The control area is located behind the testing area. All areas can be arranged on a test paper that is only made of one material. Also, different areas may be made of different materials. Each area can directly contact the liquid sample, or different areas are arranged according to the flow direction of liquid sample; and a tail end of each area is connected and overlapped with the front end of the other area. Materials used can be those with good water absorption such as filter papers, glass fibers or nitrocellulose membranes. The test paper can also be in the other forms.

The nitrocellulose membrane test strip is commonly used, that is, the testing area includes a nitrocellulose membrane (NC) on which a specific binding molecule is fixed to display the detecting result; and other test strips such as cellulose acetate membrane or nylon membrane test strips can also be used. For example, the test strips or similar apparatuses with test strips disclosed in the following patents can be applied to the testing elements or test device in this invention for analyte detection, such as the detection of the analyte in the samples: US 4857453; US 5073484; US 5119831; US 5185127; US 5275785; US 5416000; US 5504013; US 5602040; US 5622871; US 5654162; US 5656503; US 5686315; US 5766961; US 5770460; US 5916815; US 5976895; US 6248598; US 6140136; US 6187269; US 6187598; US 6228660; US 6235241; US 6306642; US 6352862; US 6372515; US 6379620, and US 6403383. The test strips and similar device provided with a test strip disclosed in the above patent literatures may be applied in the testing element or test device of the present invention for the detection of an analyte, for example, the detection of an analyte in a sample.

The test strips used in the present invention may be those what we commonly called lateral flow test strip, whose specific structure and detection principle are well known by those with ordinary skill in the art. Common test strip includes a sample collecting area or a sample application area 51, a labeled area 52, a testing area 53 and a water absorbing area 54; the sample collecting area includes a sample receiving pad, the labeled area includes a labeled pad, the water absorbing area may include a water absorbing pad; where the testing area includes necessary chemical substances for detecting the presence or absence of analyte, such as immunoreagents or enzyme chemical reagents. The nitrocellulose membrane test strip is commonly used, that is, the testing area 52 includes a nitrocellulose membrane on which specific binding molecule is fixed to display the detecting result; and other test strips such as cellulose acetate membrane or nylon membrane test strips can also be used. Of course, in the downstream of the testing area, there may also be a detecting result control area; generally, test strips appear on the control area and the testing area in the form of a horizontal line, that is a detection line 55 or a control line 56, and such test strips are conventional. Of course, they can also be other types of test strips using capillary action for detection. In addition, there are often dry chemical reagent components on the test strip, for example immobilized antibody or other reagents. When the test strip meets liquid, the liquid flows along the test strip with the capillary action, and the dry reagent components are dissolved in the liquid, then the liquid flows to the next area, the dry reagents are treated and reacted for necessary detection. The liquid flow mainly relies on the capillary action. Here, all of them can be applied to the test device of the present invention or can be disposed in contact with the liquid samples in the detection chamber or used to detect the presence or absence of analyte in the liquid samples that enter the detection chamber, or the quantity thereof.

In addition to the foregoing test strip or lateral flow test strip which is used to contact with the liquid to test whether the liquid samples contain analytes. The testing element of the present invention may be used as a test device by itself to detect an analyte in a sample. Therefore, the test device here is equal to a testing element. For example, after being mixed with the treatment solution, the fluid sample is detected with a testing element directly, specifically described as follows: when the receiving device is described to treat a fluid sample, the testing element may be used for detection alone.

### Nucleic acid

Analyte in the present invention is nucleic acid.

The term "nucleic acid" includes any compound and/or substance capable of being doped into oligonucleotide chains. Exemplary nucleic acid used according to the present invention includes but not limited to DNA; DNA includes messenger RNA (mRNA), hybrids thereof, RNAi inducer, RNAi agent, siRNA, shRNA, miRNA, antisense RNA, ribozyme, catalytic DNA, RNA formed by inducing triple helix, aptamer, vector and the like, which will be described in detail in the present application.

The term "deoxyribonucleic acid", "DNA" or "DNA molecule" refers to a molecule composed of two chains (polynucleotide), and each chain contains monomeric units, nucleotide. Nucleotides are linked with each other via a covalent bond between the glycosyl of a nucleotide and the phosphate group of a next nucleotide in the chain, thus producing an alternative glycosyl-phosphate framework. Nitrogenous bases of two separated polynucleotide chains are bound to a hydrogen bond to prepare double-stranded DNA.

The term "ribonucleic acid", "RNA", or "RNA molecule" refers to a chain connected by at least 2 base-glycosyl-phosphate groups in series. In an embodiment, the term includes compounds composed of nucleotides, of which the glycosyl part refers to ribose. In another embodiment, the terminal includes RNA with modified backbone and RNA derivatives. In an embodiment, RNA may exist in the form of transfer RNA (tRNA), small nucleolus RNAS (snRNA), ribosome RNA (rRNA), messenger RNA (mRNA), antisense RNA, small inhibitory RNA (siRNA), micro RNA (miRNA) and ribozyme. Purposes of siRNA and miRNA have been descried (Caudy A A et al, Genes & Devel16: 2491-96 and references cited therein). Moreover, these forms of RNA may be stranded by single strand chain, double strand chains, triple strand chains or quadruplex strand chains. In another embodiment, the term also includes artificial nucleic acid with other types of backbones and same bases. In another embodiment, artificial nucleic acid is PNA (peptide nucleic acid). PNA contains a peptide backbone and nucleotide bases, and can be bound to DNA and RNA molecules in another embodiment. In another embodiment, nucleotide is modified oxetane. In another embodiment, nucleotide is modified by substituting one or more phosphodiester bonds with a thiophosphate bond. In another embodiment, the modified nucleic acid contains any other variants of a phosphate backbone of a natural nucleic acid known in the art. A person skilled in the art knows the purpose and description of thiophosphate nucleic acid and PNA well, for example, Neilsen P E, CurrOpin Struct Biol 9; 353-57; [0280] and Raz N Ket al BiochemBiophys Res Commun. 297:1075-84. A person skilled in the art knows the production and use of nucleic acid well, for example, Molecular Cloning,(2001), Sambrook and Russell,eds. And Methods in Enzymology: Methods for molecular cloningin eukaryotic cells (2003) Purchio and G. C. Fareed; each nucleic acid derivative represents a separate example of the present invention.

When used herein, the term "nucleic acid" includes the following one or more types: polydeoxyribonucleotide (containing 2-deoxy-D-ribose), polyribonucleotide (containing D-ribose) and any other types of polynucleotide, which is N-glucoside of purine or pyrimidine base or modified purine or pyrimidine base (including base-free site). The term "nucleic acid", when used herein, also includes covalently bonded polymers of ribonucleotide or deoxyribonucleoside; the covalent bonding usually refers to bonding via a phosphodiester bond between subunits, but in some cases, via thiophosphate, methyl phosphonate and the like. The "nucleic acid" includes single or double-stranded DNA and single or double-stranded RNA. Exemplary nucleic acid includes but not limited to gDNA, hnRNA, mRNA, rRNA, tRNA, micro RNA (miRNA), small interfering RNA (siRNA), small nucleolus RNA (snoRNA), small nucleus RNA (snRNA) and small temporal RNA (stRNA), and any other combination thereof.

### Modified nucleotide

In some embodiments, the mRNA includes modified nucleotide; the modified nucleotide is selected from one or more of the following nucleotides: 2-aminoadenosine, 2-thiothymidine, inosine, pyrrolopyrimidine, 3-methyladenosine, 5-methylcytidine, C-5 propinyl-cytidine, C-5 propinyl-uridine, 2-aminoadenosine, C5-broxuridine, C5-floxuridine, C5-iodouridine, C5-propinyl-uridine, C5-propinyl-cytidine, C5-methylcytidine, 2-aminoadenosine, 7-denitroadenosine, 7-denitroguanosine, 8-oxyadenosine, 8-oxyguanosine, O(6)-methylguanine, pseudouridine, N-1-methyl-pseudouridine, 2-thiouridine and 2-thiocytidine, methylated base, inserted base, 2'-fluororibose, ribose, 2'-ribodesose, arabinose, hexose, thiophosphate and 5'-N-phosphoramidite bond; and the modified nucleotide described in PCT/CN2020/074825 and PCT/CN2020/106696 is modified.

### Test device

The test device refers to a device for detecting the presence or absence of an analyte. The test device for nucleic acid provided by the present invention includes a sample treatment chamber 2, a sample reaction chamber 3 and a test chamber 4 from top to bottom successively. The sample treatment chamber 2 refers to a part that receives a sample of a test device for mixing or treatment, such as, adsorption or elution of a nucleic acid adsorption membrane 9 and treatment of liquid or a liquid sample. The sample treatment chamber 2 is not present especially for receiving the test device, and may be present alone, and independently has the function of treating a fluid sample. The sample treatment chamber 2 is used to extract nucleic acid in a sample; when the sample treatment chamber 2 rotates relative to the sample reaction chamber 3, nucleic acid may be transferred into the sample reaction chamber 3 for nucleic acid amplification; the sample reaction chamber 3 is used to complete the nucleic acid amplification; when the sample reaction chamber 3 rotates relative to the test chamber 4, the amplified product may be transferred to the test chamber 4; the test chamber 4 is used for detecting the nucleic acid in the amplified product.

### Two rotations

Because there are more nucleic acid testing steps and the test device is required to be cute and convenient in volume as much as possible, the change of position relation of the sample treatment chamber 2, sample reaction chamber 3 and test chamber 4 needs to be designed delicately with the implementation of the testing process. In this way, the above three chambers are mutually matched with each other within limited space to complete nucleic acid extraction, amplification and testing, thus integrating the functions of sampling, nucleic acid purification, isothermal amplification and testing result reading by immunochromatography onto a device to prepare a simple and cute test device for nucleic acid. Further, the device can achieve nucleic acid testing only by two rotations and can ensure that each sampling or reagent adding can reach the target area in a free falling way without any extra drainage facility.

The sample reaction chamber 2 in the test device 1 provided by the present invention is used for extracting nucleic acid substances from a sample; and the sample reaction chamber 3 is used for amplifying the nucleic acid substances, thus producing an amplified product. The test chamber 4 is used for detecting the number of the amplified product or detecting the presence or absence of the amplified product.

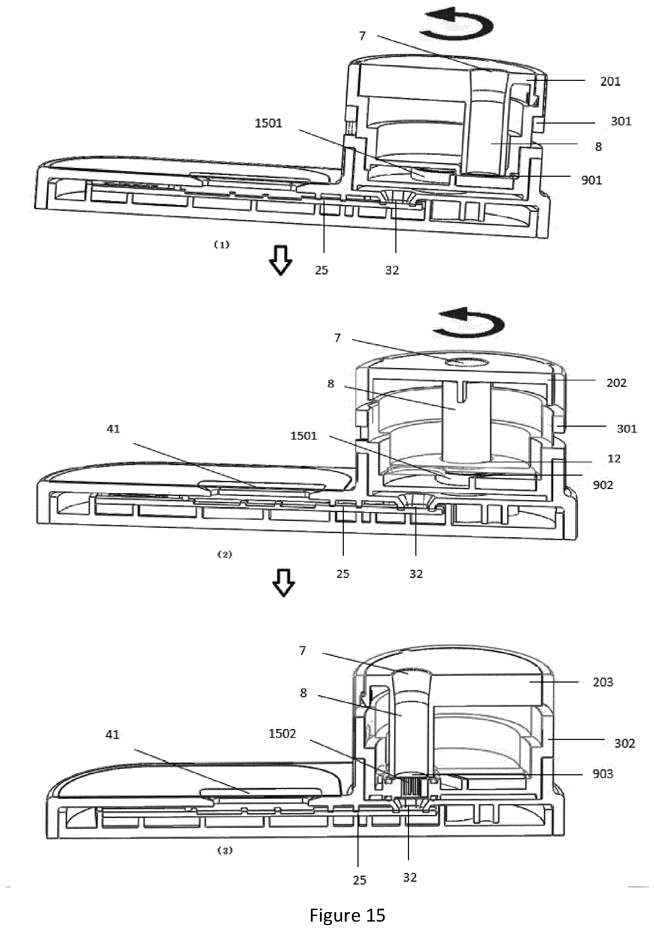

The sample treatment chamber 2 has a first position 201, a second position 202, and a third position 203 (as shown in Figure 15); meanwhile, the nucleic acid adsorption membrane 9 inside the sample treatment chamber 2 also has a first position 901, a second position 902 and a third position 903; and the nucleic acid adsorption membrane moves or rotates with the sample treatment chamber. In some embodiments, the sample reaction chamber 3 has a first position 301 and a second position 302, meanwhile, the reaction chamber 15 in the sample reaction chamber 3 also has a first position 1501 and a second position 1502.

When the sample treatment chamber 2 and the sample reaction chamber 3 are located in the first position 201, the sample treatment chamber 2, the sample reaction chamber 3 and the test chamber 4 are not in fluidic communication with each other. At this time, the sample treatment chamber is mainly used for nucleic acid lysis and analyzing nucleic acid substances from the sample for subsequent amplification. Generally, the amplification chamber has a reagent demanded by nucleic acid amplification, and the agent for sample treatment to extract nucleic acid is usually not mixed with the nucleic acid amplification reagent. Therefore, during the treatment of the sample, the treatment chamber is not in fluidic communication with the reaction chamber.

When the sample treatment chamber 2 is located in the second position 202, the sample treatment chamber 2 is in fluidic communication with the sample reaction chamber 3, and the sample reaction chamber 3 is not in fluidic communication with the test chamber 4. In case of being in the second position, the nucleic acid substances in the treatment chamber may flow into the reaction chamber, thus facilitating the subsequent amplification of the nucleic acid substances in the treatment chamber. The nucleic acid amplification herein may be isothermal amplification; the so-called isothermal amplification refers to the amplification of nucleic acid performed within a certain constant scope of temperature, for example, isothermal amplification of a recombinase or nickase; of course, PCR theory-based variable temperature amplification may be also available. Preferably, isothermal amplification is used in the present invention. Moreover, for the convenience of making nucleic acid substances in the treatment chamber flowing into, for example, the reaction chamber, at this time, the treatment chamber may continuously receive fluid reagents, and these fluid reagents elute the nucleic acid substances into the reaction chamber. Furthermore, these fluid reagents may also participate in dissolving the dry reagents preset in the reaction chamber. These reagents are necessary for nucleic acid amplification, for example, a primer, an enzyme, energy substance and the like.

When the sample treatment chamber 2 is located in the third position 203, the sample reaction chamber 3 is in fluidic communication with the test chamber 4. At this time, the reaction is completed in the reaction chamber, that is, nucleic acid amplification is completed, and the amplified product of the nucleic acid needs to flow into the test chamber for detection.

When the sample treatment chamber 2 moves to the second position 202 from the first position 201, the sample reaction chamber 3 is located in the first position 301; when the sample treatment chamber 2 moves to the third position 203 from the second position 202, the sample reaction chamber 3 is located in the second position 302, or the sample reaction chamber 3 moves to the second position 302 from the first position 301. In some embodiments, the sample treatment chamber and the sample reaction chamber move to the third position from the second position of the sample treatment chamber; the reaction chamber moves to the second position from the first position.

When the sample reaction chamber 3 is located in the second position 302, the sample reaction chamber 3 is in fluidic communication with the test chamber 4.

The sample reaction chamber 3 moves to the second position 302 from the first position 301 while the sample treatment chamber 2 moves to the third position 203 from the second position 202; or the sample treatment chamber 2 and the sample reaction chamber 3 move simultaneously, thus driving the sample treatment chamber 2 to move to the third position 203 from the second position 202 and driving the sample reaction chamber 3 move to the second position 302 from the first position 301.

The sample treatment chamber 2, the sample reaction chamber 3 and the test chamber 4 are disposed from top to bottom successively, which is cylindrical in overall appearance. Change of the different positions of the sample treatment chamber 2 and the sample reaction chamber 3 is achieved by rotation respectively. The rotation is performed by two times; the first rotation causes the sample treatment chamber 2 to move to the second position 202 from the first position 201; and the sample reaction chamber 3 and the test chamber 4 keep still; at the end of the first rotation, the sample treatment chamber 2 is in fluidic communication with the sample reaction chamber 3, and the sample reaction chamber 3 is not in fluidic communication with the test chamber 4. Therefore, the first rotation is the separate rotation of the sample treatment chamber 2; the sample reaction chamber 3 and the test chamber 4 keep still. The second rotation causes the sample treatment chamber 2 to move to the third position 203 from the second position 202; and meanwhile, the sample reaction chamber 3 moves to the second position 302 from the first position 301; at the end of the second rotation, the sample treatment chamber 2, the sample reaction chamber 3 and the test chamber 4 are in fluidic communication with each other. Therefore, the second rotation is the joint rotation of the sample treatment chamber 2 and the sample reaction chamber 3, and the test chamber 4 keeps till.

In some embodiments, the sample treatment chamber is provided with a nucleic acid adsorption membrane 9; the treatment chamber has a channel 8; one end of the channel is communicated with the outside, and another end of the channel is covered by the membrane such that the sample and lysis solution are added to the channel 8; nucleic acid substances are adsorbed by the membrane, and other liquid flows across the membrane into a waste reservoir.

### DETAILED DESCRIPTION OF THE INVENTION

The present invention will be further described with reference to the examples hereafter. It needs to be indicated that the following examples are aimed at facilitating the understanding of the present invention, but not construed as limiting the protection scope of the present invention. Reagents not particularly specified in the examples are products known in the art and can be commercially available.

### Example 1 Test device for nucleic acid provided in the present invention

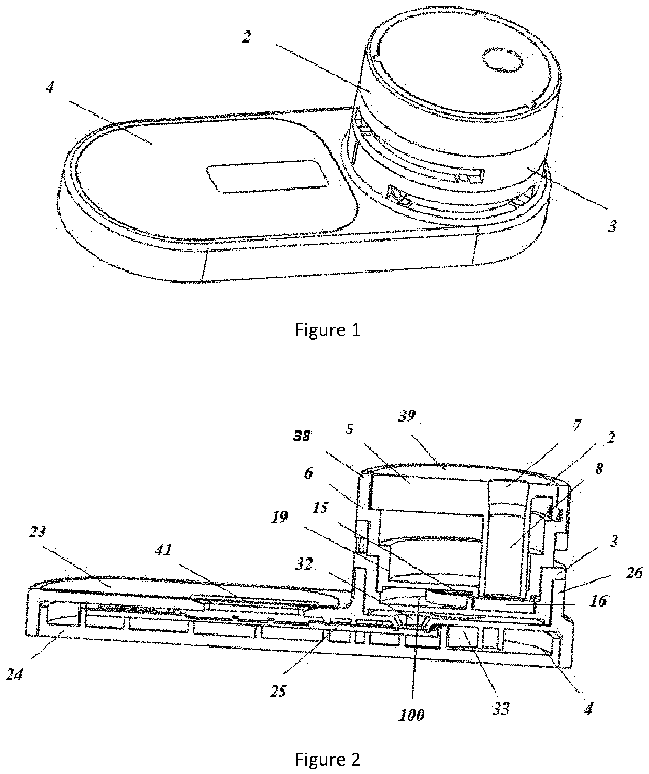

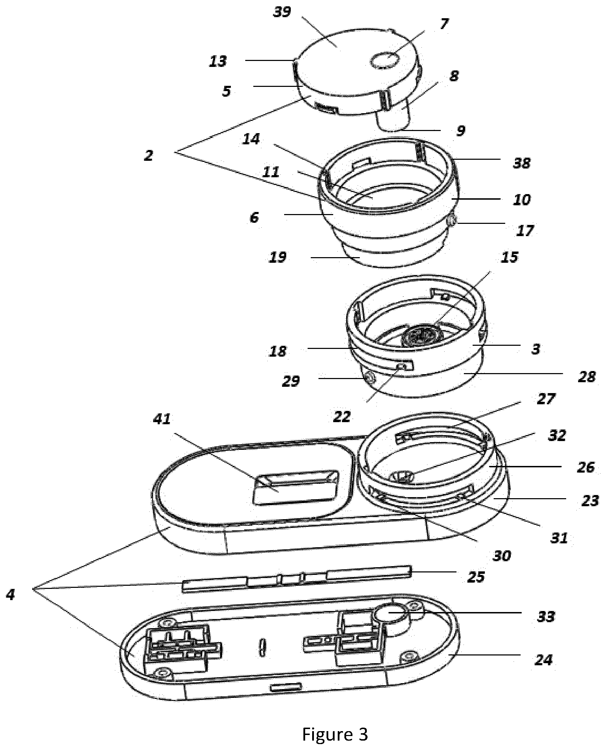

The test device for nucleic acid provided in this example is shown in Figures 1-14.

In some embodiments, as shown in Figures 1-3, the test device for nucleic acid 1 provided in this example includes a sample treatment chamber 2, a sample reaction chamber 3 and a test chamber 4 from top to bottom successively. The sample treatment chamber 2 is used to extract or separate nucleic acid in a sample; when the sample treatment chamber 2 rotates relative to the sample reaction chamber 3, nucleic acid may be transferred into the sample reaction chamber 3 for nucleic acid amplification; the sample reaction chamber 3 is used to complete the nucleic acid amplification; when the sample reaction chamber 3 rotates relative to the test chamber 4, the amplified product may be transferred to the test chamber 4; the test chamber 4 is used for detecting the nucleic acid in the amplified product.

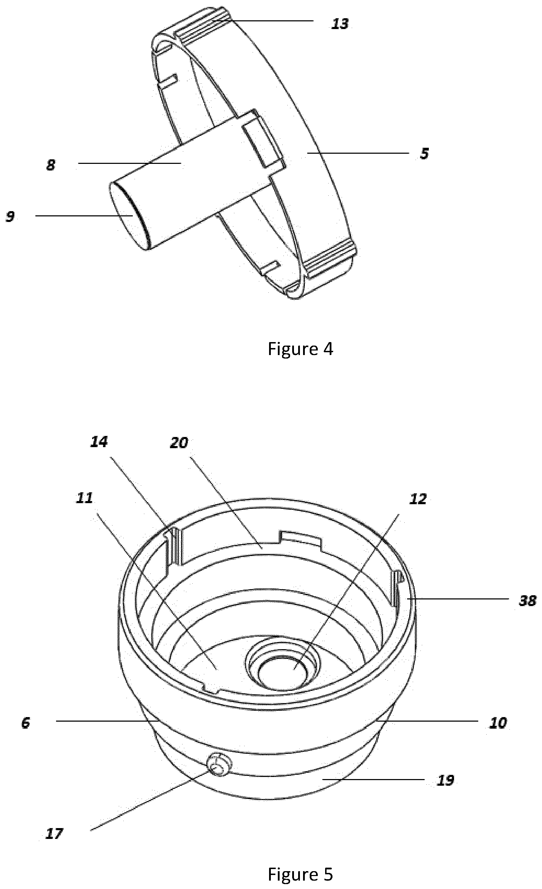

In some embodiments, as shown in Figures 4 and 5, the sample treatment chamber 2 is used to extract nucleic acid in a sample, and includes an top cover 5 and a bottom cover 6; the top cover 5 is provided with a sample inlet 7; a sampling channel 8 is connected below the sample inlet 7; a nucleic acid adsorption membrane 9 is fixed at the bottom of the sampling channel 8, and the nucleic acid adsorption membrane 9 is used for adsorbing nucleic acid in the sample; the bottom cover 6 includes an outer wall 10 and a bottom surface 11; the bottom surface 11 is provided with a c 12; the top cover 5 is fixed with the bottom cover 6, and the through hole 12 is always aligned at the sampling channel 8.

In some embodiments, as shown in Figures 4-7, a bulge 13 is disposed on the periphery of the side wall of the top cover 5; the bottom cover 6 is provided with a groove 14 in fit with the bulge 13 on the side wall; the top cover 5 and the bottom cover 6 are respectively fixed via the bulge 13 and the groove 14, that is, the top cover 5 and the bottom cover 6 have fixed positions; the through hole 12 on the bottom surface of the bottom cover 6 is always aligned at the sampling channel 8; the top cover 5 and the bottom cover 6 are only rotated together instead of separate rotation.

In some embodiments, the side wall of the top cover 5 has a diameter slightly smaller than that of the bottom cover 6 such that the top cover 5 may be sleeved into the side wall of the bottom cover 6; a bulge loop 20 is disposed on the side wall of the bottom cover 6 such that the top cover upper plane 39 is just parallel to the highest section 38 of the side wall of the bottom cover after the top cover 5 is combined with the bottom cover 6.

In some embodiments, the sample inlet 7 has a diameter of 8 mm, and the sampling channel 8 is cylindrical, and the bottom surface has a diameter the same as the sample inlet 7, and the height is 19 mm, extending downwards directly from the sample inlet 7; the sample reaches to the nucleic acid adsorption membrane 9 from the sample inlet 7 via the sampling channel 8. The nucleic acid adsorption membrane 9 is a circular film and may be selected from a silicon dioxide film or polysiloxane (silica) film; and the diameter thereof is consistent with or slightly greater than the diameter of the sample inlet 7. The nucleic acid adsorption membrane 9 is fixed at the bottom of the sampling channel 8, and is required to fully cover the outlet at the bottom of the sampling channel 8; the sample enters from the sample inlet 7 and is capable of impacting the nucleic acid adsorption membrane 9 with greater force after being accelerated by a certain height of sampling channel 8, which promotes the nucleic acid adsorption membrane 9 to adsorb the nucleic acid in the sample more fully, thereby improving the detection sensitivity of nucleic acid. Therefore, the whole sample flows through the nucleic acid adsorption membrane 9 after flowing from the sample inlet 7, and then almost all the nucleic acid in the sample is absorbed by the nucleic acid adsorption membrane 9.

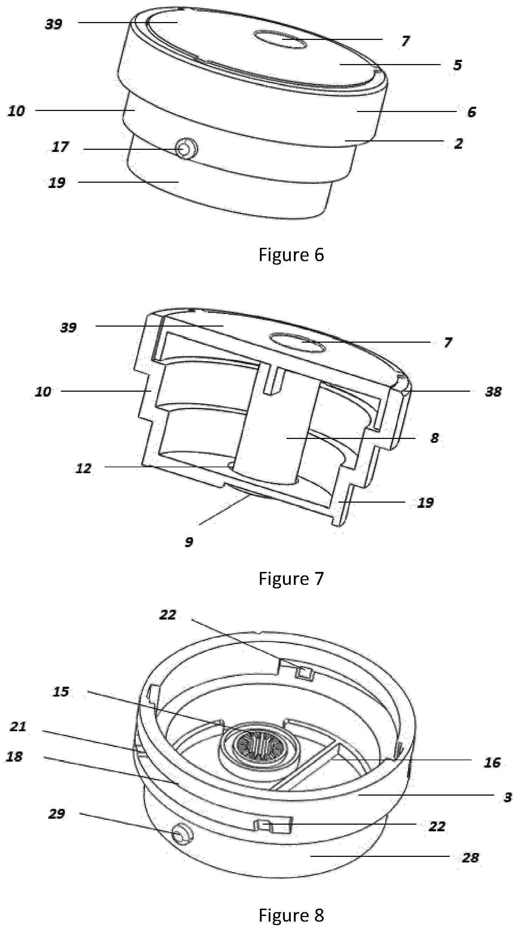

In some embodiments, as shown in Figure 8, a reaction chamber 15 and a filter paper storage slot 16 are disposed at the bottom of the sample reaction chamber 3; the filter paper storage slot 16 is provided with filter paper used for adsorbing the excessive sample, for example, the sample remaining after flowing through the membrane, and the sample herein is generally mixed with the reagent for nucleic acid lysis. The reaction chamber 15 is provided with a nucleic acid amplification membrane or fixed with a nucleic acid amplification drying agent. Such a configuration mode is to make the testing process more convenient and flexible, thus avoiding repeated addition of multiple reagents from the sample inlet 7 during the detection and preventing the reagent from being adsorbed by the nucleic acid adsorption membrane 9 at the sample inlet 7 to affect the isothermal amplification, leading to detection errors. Therefore, when the treatment chamber is located in the first position, the nucleic acid separation membrane of the treatment chamber is located just below of the test paper storage slot 16 and is spaced from the reaction chamber. For example, as shown in Figure 8, the reaction chamber is located at different planes with the test paper storage slot, and there is a straight-line distance. Moreover, a partition part is disposed between the test paper storage slot 16 and the reaction chamber 15 such that there is no fluidic communication in another region.

In some embodiments, as shown in Figure 9, a first rotary buckle 17 is disposed on the outer wall 10 of the bottom cover 6 of the sample treatment chamber 2, and a first rotary slot 18 is disposed above the side wall of the sample reaction chamber 3; when the sample treatment chamber 2 rotates relative to the sample reaction chamber 3, the first rotary buckle 17 moves from one end of the first rotary slot 18 to another end thereof; the number of the first rotary buckle 17 and the number of the first rotary slot 18 are more than one.

In some embodiments, as shown in Figure 5, the sample treatment chamber 2 has a narrowed diameter at the lower end 19 of the side wall of the bottom cover 6 such that the side wall of the bottom cover 6 may be sleeved into the side wall of the sample reaction chamber 3 (as shown in Figure 8). Therefore, after being combined together, the sample treatment chamber 2 and the sample reaction chamber 3 form a regular cylinder which is more beautiful and also more convenient to rotation.

In some embodiments, 2 bulging first rotary buckles 17 are disposed on the periphery of the lower end 19 of the side wall of the bottom cover 6, and are symmetrically distributed at the lower end 19 of the outer wall; 2 first rotary slots 18 are disposed at the top of the outer wall of the sample reaction chamber 3, and are symmetrically distributed at the top of the outer wall; the first rotary slot 18 is a strip-type transverse hollow groove disposed on the other wall; when the lower end 19 of the side wall of the bottom cover is sleeved into the side wall of the sample reaction chamber 3, the bulging first rotary buckle 17 is just located in the hollow first rotary slot 18.

In some embodiments, as shown in Figure 8, both left end and right end of the first rotary slot 18 are respectively provided with bulged limiting blocks. In the initial position, the first rotary buckle 17 is located at the leftmost end of the first rotary slot 18, blocked and fixed by the limiting block 21; at the end of nucleic acid extraction (after sampling and the nucleic acid in the sample is absorbed by the nucleic acid adsorption membrane), first rotation is performed; the first rotary buckle 17 needs to overcome the blocking of the left-end limiting block 21 to slide rightwards along with the first rotary slot 18 until the first rotary buckle 17 overcomes the right-end limiting block 22 to enter into the rightmost end and be blocked and fixed by the limiting block 22 and may not continue to rotate rightwards; at this time, the sampling channel 8 and the nucleic acid adsorption membrane 9 are just transferred above the amplification chamber 15 and ready for the nucleic acid washing and isothermal amplification. In some embodiments, the membrane adsorbed with nucleic acid is just covered above the reaction chamber. In this way, when the nucleic acid substances on the membrane are eluted by liquid, the nucleic acid substances may flow into the reaction chamber smoothly.

As shown in Figure 9, the test chamber 4 includes a test strip top cover 23 and a test strip bottom cover 24; the transverse flow test strip 25 is fixed in the test strip top cover 23 and the test strip bottom cover 24, used for detecting the nucleic acid in the amplified product. The test strip top cover 24 is provided with a sampling hole 32 and a test result observation window 41; the sampling hole 32 is aligned at the sampling test position of the test strip; the test result observation window 41 is aligned at a test result reading window of the test strip, used for reading the test result. A cylindrical groove 26 is disposed in the test strip top cover 23; the sampling hole 32 is located on the bottom surface of the cylindrical groove 26; and a second rotary slot 27 is disposed on the groove wall; a second rotary buckle 29 is disposed at the lower portion 28 of the outer wall of the sample reaction chamber 3; when the sample reaction chamber 3 rotates relative to the test chamber 4, the second rotary buckle 29 moves from one end of the second rotary slot 27 to another end thereof. The number of the second rotary buckle 29 and the number of the second rotary slot 27 are more than two. two second rotary buckles 29 are symmetrically distributed at the lower end 28 of the outer wall of the sample reaction chamber 3; and two second rotary slots 27 are symmetrically distributed on the wall of the cylindrical groove 26. The second rotary slot 27 is a strip-type transverse hollow groove disposed on the groove wall; when the lower end 28 of the outer wall of the sample reaction chamber 3 is sleeved into the cylindrical groove 26, the bulging second rotary buckle 29 is just located in the hollow second rotary slot 27. Both left end and right end of the second rotary slot 27 are respectively provided with bulged limiting blocks. In the process of first rotation, the second rotary buckle 29 and the second rotary slot 27 always keep still in position; the second rotary buckle 29 is located at the leftmost end of the second rotary slot 27, blocked and fixed by the left-end limiting block 30; at the end of nucleic acid amplification, second rotation is performed; the second rotary buckle 29 needs to overcome the blocking of the left-end limiting block 30 to slide rightwards along with the second rotary slot 27 until the second rotary buckle 29 overcomes the right-end limiting block 31 to enter into the rightmost end and be blocked and fixed by the limiting block 31; at this time, the nucleic acid adsorption membrane 9 and the reaction chamber 15 are jointly transferred above the sampling hole 32 of the test strip 25.

In some embodiments, as shown in Figure 9, the heating hole 33 is located on the test strip bottom cover 24; and the heating hole 33 is located below the reaction chamber 15, used for supplying a heat source for the nucleic acid amplification. The heat source for the nucleic acid amplification may be supplied by configuring a heating hole 33; the heating hole 33 is matched with a constant-temperature heating equipment, and may be placed on the circular metal bulge from any constant-temperature heat source; the constant-temperature heating equipment supplies a heat source for the reaction chamber 15 via the heating hole 33, thus starting and maintaining the isothermal amplification. Of course, the heat source of the isothermal amplification of nucleic acid may be also directly disposed in the inner part of the test device for nucleic acid 1. In this way, it is unnecessary to further supply a constant-temperature heating equipment.

In some embodiments, the structure of the transverse flow test strip 25 is shown in Figure 10, including a sample application area 51, a labeled area 52, a testing area 53, and a water absorbing area 54; the testing area 53 contains a detection line 55 or a control line 56. In this example, the labeled area 52 is coated with anti-FAM antibody labeled colloidal gold; the detection line 55 is coated with streptavidin, and the control line 56 is coated with goat-anti-mouse antibody (the antibody of goat-anti-mouse IgG). The principle of nucleic acid testing is as follows: the amplification primer contains biotin modification, and the 5' terminal in the probe is modified by FAM fluorophores; after being amplified by the primer probe, the 5' terminal of the amplified product carries the FAM fluorophores, and the 3' terminal carries the biotin group, and then the target sequence may be smoothly detected by the transverse flow test strip 25.

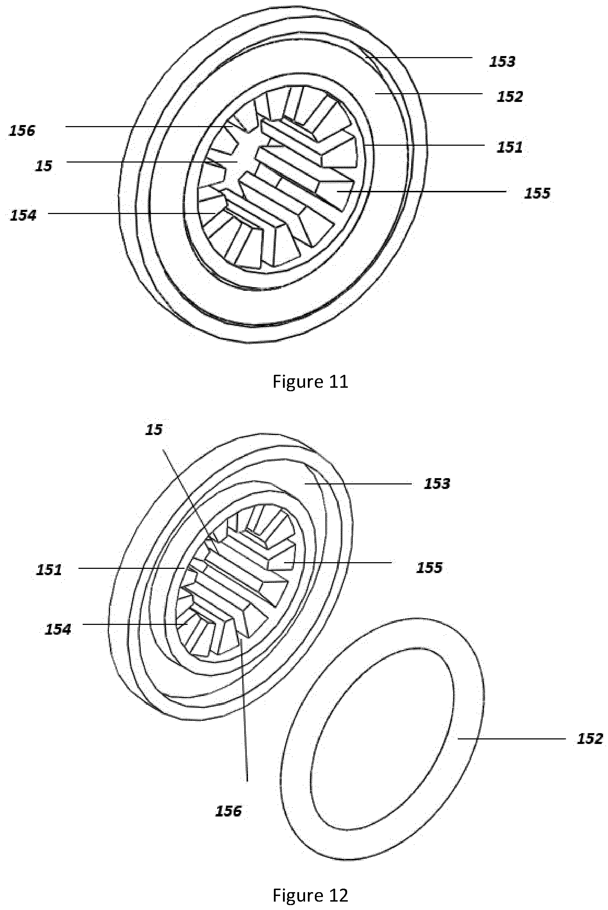

In some embodiments, as shown in Figure 11, the reaction chamber 15 is a cylindrical cavity and cut through from top to bottom; there is neither an upper bottom surface, nor a lower bottom surface. No upper bottom surface is convenient to directly wash the sample into the reaction chamber 15 from the upper nucleic acid adsorption membrane 9; and no lower bottom surface is convenient to transfer the amplified product in the reaction chamber 15 onto the sampling hole 32; at this time, liquid in the reaction chamber 15 may smoothly drip onto the test strip 25 for nucleic acid due to being lack of the support from the bottom board. A sealing gasket 152 is disposed at the periphery of the lower end 151 of the cylindrical chamber 15; when the reaction chamber 15 slides on the bottom surface 100 of the cylindrical groove 26, the reaction chamber is located on the bottom surface and liquid in the reaction chamber 15 is free of spilling over the reaction chamber 15 under the protection of the gasket 152; when the reaction chamber 15 rotates above the sampling hole 32, the amplified product drips onto the test strip from the sampling hole 32 in a free falling way for nucleic acid testing. Therefore, when the reaction chamber is located on the bottom surface 100 of the plane; the bottom surface of the reaction chamber is equivalent to the bottom surface of the cylindrical groove; the sealing gasket 152 contacts the bottom surface; liquid or reagent in the reaction chamber is located in the sealing gasket. In this way, one side of the bottom surface 100 is the reaction chamber, and another side thereof may be used for heating such that the amplification is performed in the reaction within multifunctional temperature.

In some embodiments, the reaction chamber 15 has no lower bottom surface, but is disposed on the test strip bottom cover 24; the gasket 152 ensures that liquid in the reaction chamber 15 will not leak away from the gap of the lower end 151 on the side wall of the reaction chamber and from the surface of the test strip bottom cover 24; moreover, when the second rotation is performed at the end of the amplification, the reaction chamber 15 will slide on the surface of the test strip bottom cover 24; with the protection of the gasket 152, the sliding may not cause the reaction solution to leak away from the reaction chamber 15.

In some embodiments, as shown in Figure 12, a gasket groove 153 is disposed on the periphery of the lower end 151 of the reaction chamber, used for fixing the position of the gasket 152. In this way, the movement of the reaction chamber drives the sealing gasket 152 to move together. In this way, the bottom surface of the reaction chamber has different sealing/unsealing states at different positions; in the process of reaction, the bottom surface of the reaction chamber may be at least free of leakage, while solution in the reaction chamber is required to flow into the test chamber, the bottom surface 100 is not required, but removed from the bottom of the reaction chamber. The way of removing is rotation to make the position of the reaction chamber changed.

In some embodiments, as shown in Figure 11, a layer of drainage groove 154 is disposed on the inner wall of the reaction chamber 15; the drainage groove 154 is composed of multiple trapezoid columns 155 spaced equally which are disposed on the inner wall; a drainage channel 156 is formed between the adjacent two trapezoid columns 155. Since there is a trace amount of sample for nucleic acid amplification, how to ensure the drainage of the full sample into the reaction chamber 15 is a very crucial step to guarantee the detection sensitivity of nucleic acid. Drainage grooves 154 are disposed around the inner walls of the reaction chamber 15, which may drain the sample to be tested into the reaction chamber 15 to the maximum extent, thus avoiding leakage. At this time, nucleic acid substances are absorbed by a membrane in the present invention. When the nucleic acid substances need to be eluted from the membrane, the eluant will flow across the membrane to flow into the reaction chamber. To make the eluant flowing into the reaction chamber better, the gap of the drainage groove should be very small, and liquid flows into the reaction chamber from the membrane under capillary action.

Preferably, the trapezoid columns 155 are arranged directly from the upper end of the reaction chamber 15 to the lower end thereof such that the sample may be drained to the lower end from the upper end of the reaction chamber 15 all the time. This is mainly because an immobilization membrane or a drying agent is placed at the lower end of the reaction chamber 15; only when the sample is drained to the lower end from the upper end of the reaction chamber 15, the sample may be ensured to fully contact with the immobilization membrane or drying agent, thus improving the amplification efficiency.

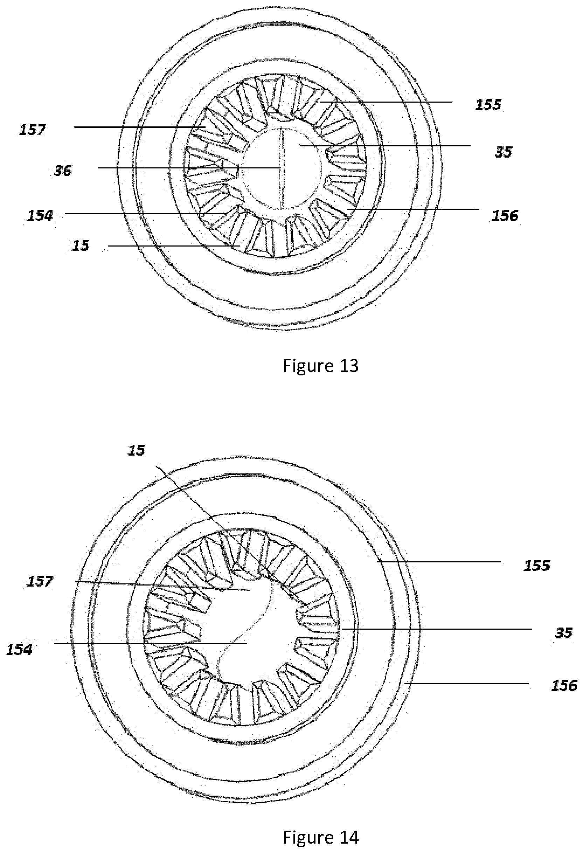

Preferably, as shown in Figure 13, an upward-narrowing step 157 is disposed on the upper end of each trapezoid column 155; the drainage channel 156 has a width of 0.1-0.5 mm. The upward-narrowing step 157 at the upper end of the trapezoid column 155 may contact with the upper nucleic acid adsorption membrane 9, thus assisting in absorbing the sample from the upper part into the drainage channel 156, thus achieving full drainage. In this example, the upward-narrowing step 157 has a height of 0.2 mm. The drainage channel 156 formed between each trapezoid column 155 has a consistent width; the narrower the width of the drainage channel 156 is, the better the drainage effect is. However, too narrow drainage channel 156 will increase the difficulty of the manufacturing process. Therefore, it needs to choose a proper width of the drainage channel.

Preferably, the number of the trapezoid columns 155 is 13, and the number of the drainage channels 156 is 13; the drainage channels are distributed evenly along with the inner wall of the reaction chamber 15, and the width of each drainage channel 0.5 mm; the trapezoid column 155 has a bottom width of 0.9 mm, and the distance protruding from the inner wall of the reaction chamber 15 to the outside is 1.25 mm and the height is 3.7 mm. The configuration of width and number of the drainage channel 156 (the number of the drainage channel determines the number and arrangement of the trapezoid columns, thus determining the width of each trapezoid column) determines whether the drainage effect can be improved to the maximum extent or not. The cylinder of the reaction chamber 15 in this example has a cross section diameter of 6 mm. A great number of studieshave shown that when the width of the drainage channel 156 is 0.5 mm and the trapezoid column 155 has a bottom width of 0.9 mm, and the distance protruding from the inner wall of the reaction chamber 15 to the outside is 1.25 mm, a better drainage effect may be achieved.

In some embodiments, as shown in Figure 13, when a nucleic acid amplification membrane is placed in the reaction chamber 15 in advance, the nucleic acid amplification membrane includes a first reaction membrane 35 and a second reaction membrane 36. These membranes are pretreated with a necessary reagent for nucleic acid amplification. The recombinase reagent and the PEG buffer reagent in the amplification reagent must be placed separately before the amplification, otherwise the recombinase is easily wrapped by PEG, thus affecting the amplification efficiency. Therefore, two layers of immobilized reagent reaction membranes (the first reaction membrane 35 and the second reaction membrane 36) need to be configured. There are three ways of arrangement to the first reaction membrane 35 and the second reaction membrane 36: 1, the first reaction membrane 35 is laid on the lower end of the reaction chamber 15; the second reaction membrane 36 and the first reaction membrane 35 are placed horizontally; 2, the second reaction membrane 36 is vertically placed above the first reaction membrane 35; 3, the second reaction membrane 36 and the first reaction membrane 35 are placed vertically. In this example, the second way is adopted preferably, namely, the first reaction membrane 35 is laid on the lower end of the reaction chamber 15, and the second reaction membrane 36 is located at the central position of the cylinder of the reaction chamber 15 and vertically placed above the first reaction membrane 35. The reason why the second way is adopted preferably is as follows: experimental results show that the arrangement position of the two layers of reaction membranes will also affect the drainage effect of the sample; when the second reaction membrane 36 is vertically placed above the first reaction membrane 35 and located at the central position, the nucleic acid adsorption membrane may be contacted, which will assist improving the drainage effect, thus improving the amplification efficiency and improving the detection sensitivity of nucleic acid. The nucleic acid reagent reaction membrane herein is a reagent necessary for nucleic acid amplification. A function of the reagent is to make liquid dissolved rapidly to form an amplification system after flowing into the reaction chamber, and another function is drainage. It may be understood that the drainage groove 154 and the drainage membrane (containing a reagent for nucleic acid amplification) in the reaction chamber of the present invention may be exist together or exists alone.

Of course, the reaction chamber 15 may be fixed with nucleic acid amplification drying agents, respectively a first drying agent and a second drying agent; the first drying agent and the second drying agent are spaced by a division bar 37 disposed at the bottom surface of the reaction chamber (Figure 14). The first drying agent is a recombinase reagent required by nucleic acid amplification; the second drying agent is a PEG buffer reagent required by nucleic acid amplification; the first drying agent and the second drying agent are respectively disposed at both sides of the division bar 37 first, and dried or frozen-dried to a drying agent; during detection, an eluant is added such that the two reagents are redissolved and mixed for nucleic acid amplification. Therefore, the division bar 37 may be not too high to avoid mixing difficulty after being redissolved. In this example, the division bar 37 has a height of 2 mm.

### Example 2 Rotation and change of state of the test device for nucleic acid provided in the present invention

The test device for nucleic acid 1 provided in the example may complete nucleic acid amplification and testing in the way of two rotations. The rotation and change of state are shown in Figure 15, where Figure 15 (1) shows an initial state, and Figure 15 (2) shows a state after first rotation, and Figure 15 (3) shows a state after second rotation.

As can be seen from Figure 15, the sample treatment chamber 2 has a first position 201, a second position 202, and a third position 203; meanwhile, the nucleic acid adsorption membrane 9 inside the sample treatment chamber 2 also has a first position 901, a second position 902 and a third position 903; the sample reaction chamber 3 has a first position 301 and a second position 302; meanwhile, the reaction chamber 15 in the sample reaction chamber 3 also has a first position 1501 and a second position 1502.

The test device for nucleic acid 1 is located in the initial position when not used (Figure 15 (1)); that is, the sample treatment chamber 2 is located in the first position 201 before rotation and the nucleic acid adsorption membrane 9 is also located in the first position 901; the sample reaction chamber 3 is located in the first position 301 and the reaction chamber 15 is also located in the first position 1501. At this time, the sample treatment chamber 2, the sample reaction chamber 3 and the test chamber 4 are not in fluidic communication with each other. That is, the sampling channel 8 of the sample treatment chamber 2, the reaction chamber 15 of the sample reaction chamber 3 and the sampling hole 32 of the test chamber 4 are not in fluidic communication with each other. However, at this time, the sampling channel 8 is located above the test paper storage slot 16; the sampling channel 8, the through hole 12 and the test paper storage slot 16 are vertically distributed in a straight line from top to bottom. Therefore, sampling may be started at the initial position, and the sample is added vertically from the sample inlet 7 and contacts the nucleic acid adsorption membrane 9 after flowing through the sampling channel 8 first, and then nucleic acid in the sample is adsorbed by the nucleic acid adsorption membrane 9, and the remaining liquid flows into the test paper storage slot 16 after permeating the nucleic acid adsorption membrane 9, thus being adsorbed by test paper in the test paper storage slot 16.

After the first rotation (Figure 15 (2)), the sample treatment chamber 2 rotates to the second position 202 from the first position 201, and the nucleic acid adsorption membrane 9 in the sample treatment chamber 2 also rotates from the first position 901 to the second position 902, while the sample reaction chamber 3 is still located in the first position 301, and the reaction chamber 15 is still located in the first position 1501. At this time, the sample treatment chamber 2 is in fluidic communication with the sample reaction chamber 3, and the sample reaction chamber 3 is not in fluidic communication with the test chamber 4. That is, the sampling channel 8 of the sample treatment chamber 2 rotates above the reaction chamber 15 of the sample reaction chamber 3 such that the sampling channel 8 is in fluidic communication with the reaction chamber 15; when the reaction chamber 15 has no movement, the reaction chamber 15 is not in fluidic communication with the sampling hole 32 of the test chamber 4.

The first position 901 of the nucleic acid adsorption membrane 9 is a position where the nucleic acid adsorption membrane 9 is located above the test paper storage slot 16; the second position 902 is a position where the nucleic acid adsorption membrane 9 is located above the reaction chamber 15; when the first rotary buckle 17 is located at one end of the first rotary slot 18, the nucleic acid adsorption membrane 9 is located in the first position 901; in case of the first rotation, the first rotary buckle 17 moves to another end of the first rotary slot 18, and the nucleic acid adsorption membrane 9 is transferred to the second position 902. At the end of the first rotation, the nucleic acid adsorption membrane 9 is located in the second position 902; at this time, the sampling channel 8, the through hole 12 and the reaction chamber 15 are vertically distributed in a straight line, and it may begin to add the eluant and to perform the isothermal amplification of nucleic acid.

The first rotation causes the nucleic acid adsorption membrane 9 adsorbed with nucleic acid to be transferred above the amplification chamber 15; at this time, the eluant may be added from the sample inlet 7, and the eluant flows into the sampling channel 8 in a free falling way to elute the nucleic acid on the nucleic acid adsorption membrane 9 into the reaction chamber 15 with an eluant at the shortest distance, and then isothermal amplification may be performed in the reaction chamber 15.

At the end of the second rotation (Figure 15 (3)), the sample treatment chamber 2 moves to the third position 203 from the second position 202, and the nucleic acid adsorption membrane 9 inside the sample treatment chamber 2 also rotates to the third position 903 from the second position 902; the sample reaction chamber 3 moves to the second position 302 from the first position 301, and the reaction chamber 15 also moves to the second position 1502 from the first position 1501. At this time, the sample reaction chamber 3 is in fluidic communication with the test chamber 4, that is, the reaction chamber 15 of the sample reaction chamber 3 is located above the sampling hole 32 of the test chamber 4; meanwhile, the sample treatment chamber 2 is also in fluidic communication with the sample reaction chamber 3 and the test chamber 4. That is, the sampling channel 8 of the sample treatment chamber 2 is located above the reaction chamber 15; the sampling channel 8, the though hole 12, the reaction chamber 15 and the heating hole 32 are vertically distributed in a straight line.

It may be understood that the second rotation refers to a process of joint rotation of the sample treatment chamber 2 and the sample reaction chamber 3; the sample reaction chamber 3 rotates to the second position 302 from the first position 301, thus driving the sample treatment chamber 2 to rotate to the third position 203 from the second position 202. The first position 901 and the second position 902 of the nucleic acid adsorption membrane 9 are relative to the sample reaction chamber 3; in case of the second rotation, the nucleic acid adsorption membrane 9 moves together with the sample reaction chamber 3. Therefore, the nucleic acid adsorption membrane 9 has no change of position relative to the sample reaction chamber 3; that is, when the sample reaction chamber 3 rotates relative to the test chamber 4, the sample treatment chamber 2 rotates together with the sample reaction chamber 3, and the nucleic acid adsorption membrane 9 is always located above the reaction chamber 15. However, relative to the test chamber 4, the nucleic acid adsorption membrane 9 further has a third position 903 because at the end of the second rotation, the nucleic acid adsorption membrane 9 and the reaction chamber 15 are together transferred above the sampling hole 32 of the test chamber 4.

The second rotation causes the reaction chamber 15 to have a first position 1501 and a second position 1502; the first position refers that the reaction chamber 15 is located above the heating hole 33; the second position refers that the reaction chamber 15 is located above the sampling hole 32. When the second rotary buckle 29 is located at one end of the second rotary slot 27, the reaction chamber 15 is located in the first position 1501; when the second rotary buckle 29 moves to another end of the second rotary slot 27, the reaction chamber 15 is located in the second position 1502.

The reaction chamber 15 is always located in the first position 1501 from when the test device for nucleic acid 1 is located in the initial position until the completion of the first rotation. This is because the first rotation only refers to the rotation of the sample treatment chamber 2, and the sample reaction chamber 3 keeps still. During the second rotation, the sample treatment chamber 2 rotates together with the sample reaction chamber 3 such that the reaction chamber 15 rotates above the sampling hole 32 from the upper part of the heating hole 33, thus achieving sampling detection.

The second rotation causes the reaction chamber 15 together with the amplified product to be dripped onto the test strip 25 via the sampling hole 32; the amplified product drips onto the test strip 25 in a free falling way for detection, which is more convenient and flexible without any error in use. Therefore, the present invention ensures that each sampling or reagent adding can reach the target area in a free falling way by designing two rotations. Moreover, the present invention has a compact and small structure without any extra drainage facility, which is capable of improving efficiency and detection sensitivity.

The test device for nucleic acid 1 provided in this example may complete the nucleic acid sample testing by two simple operations only; the first rotation is to fix the test device for nucleic acid 1 with one hand and to hold the upper end (e.g., outer wall of the sample treatment chamber 2) of the test device for nucleic acid 1 with another hand for rotation, which is a kind of rotation of the sample treatment chamber 2 relative to the sample reaction chamber 3; namely, the sample treatment chamber 2 rotates separately, while the sample reaction chamber 3 and the test chamber 4 keep still. The second rotation is to fix the test device for nucleic acid 1 with one hand and to hold the middle (e.g., outer wall of the sample reaction chamber 3) or upper position of the test device for nucleic acid 1 with another hand for rotation, which is a kind of rotation of the sample reaction chamber 3 relative to the test chamber 4; namely, the sample treatment chamber 2 rotates together with the sample reaction chamber 3, while the test chamber 4 keeps still. Since rotation may not be performed continuously after rotation for once, the order of the two rotations will be free of error and free of misoperation.

### Example 3 Test system for nucleic acid provided in the present invention

The test system for nucleic acid provided in this example includes a test device for nucleic acid provided in Example 1 and supporting nucleic acid testing reagents. The nucleic acid testing reagents include a lysis solution, an eluant and a nucleic acid amplification reagent; the nucleic acid adsorption membrane in the test device for nucleic acid is made of a polysiloxane silica membrane (Shenzhen Biocomma, Y-SM-BC-1).

### 1. Preparation of the lysis solution

Formula of the lysis solution: 0.1 M Tris, 0.2 M ethylene diamine tetraacetic acid (EDTA), 3 M guanidinium isothiocyanate and 5% Triton 100;
5% Triton-100, 50 mmol Tris-HCL and 50 mmol NaOH.
2 ml lysis solution was taken and pre-added to a reagent bottle; the size of the reagent bottleneck is matched with the size of the sample inlet (without cover) of the test device for nucleic acid to be sealed and matched with each other.

### 2. Preparation of the nucleic acid amplification reagent

A dry immobilized nucleic acid amplification reagent was placed in the test device for nucleic acid of the example in advance, mainly including two types of (1) immobilization membrane or (2) drying agent.
(1) Immobilization membrane: glass fiber membrane (model: 8860), including a first reaction membrane and a second reaction membrane; size: a diameter of 6 mm and a thickness of 1 mm.

### Preparation of the first reaction membrane:

Preparation of the first reaction liquid: 30 mM Tris-acetic acid buffer solution with a pH value of 8.0, 50 mM potassium acetate, 3 mM dithiothreitol, 2 mM ATP, 20 mM phosphocreatine, 100 ng/µl creatine kinase, 600 ng/µl *Escherichia coli* SSB protein, 150 ng/µl phage uvsX protein, 25 ng/µl phage uvsY protein, 80 ng/µl klenow polymerase klenow fragment (exo-), 50 ng/µl exonuclease III, 200U reverse transcriptase, 450 µM dNTP, 420 nM of each forward primer, 420 nM of each reverse primer and 120 nM of each fluorescent probe.

0.02 ml first reaction liquid was taken and fixed on the first reaction membrane, and dried at 50°C.

### Preparation of the second reaction membrane:

Preparation of the second reaction membrane: 5% polyethylene glycol (molecular weight: 20000) and 0.28 M magnesium acetate.

0.02 ml second reaction liquid was taken and fixed on the second reaction membrane, and dried at 50°C.
(2) Drying agent: including a first drying agent and a second drying agent.

The first drying agent contains components and the second drying agent contains components.

The method for preparing the first drying agent and the second drying agent in the reaction chamber in advance is as follows: reagents contained were respectively added on both sides of the division bar at the bottom of the reaction chamber, then dried or frozen-dried to prepare the test device for nucleic acid added with the first drying agent (the first reaction liquid) and the second drying agent (the second reaction liquid) in advance.

### 3. Preparation of the eluant

Formula of the eluant: 0.01 M Tris, 0.001 M ethylene diamine tetraacetic acid (EDTA), and 0.28 M magnesium acetate.

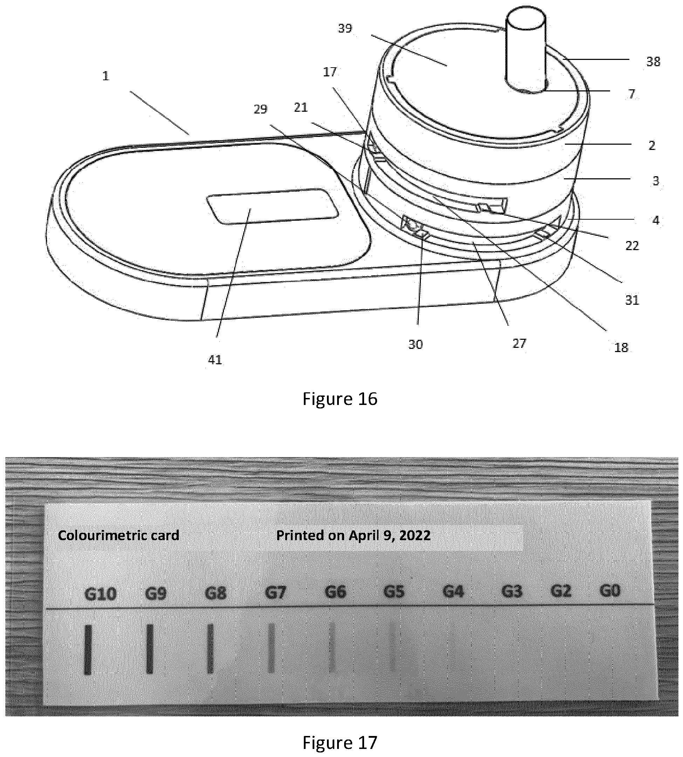

0.06 ml eluant was taken and added to a reagent bottle in advance. The sample inlet of the test device for nucleic acid is not provided with a cover, and the reagent bottleneck may be sealed and matched with the sample inlet. When the eluant is added by the reagent bottle, the reagent bottleneck may be directly screwed with the sample inlet and may not be taken out any more (Figure 16), which plays the roles of sealing and anti-pollution during the nucleic acid amplification and testing process; and these are subjected to bio-waste treatment together after the detection, thus preventing biological contamination.

In this example, the nucleic acid testing is performed by a test system for nucleic acid; the dried nucleic acid amplification reagent is fixed by the first reaction membrane and the second reaction membrane. Moreover, the first reaction membrane is laid on the lower end of the reaction chamber and the second reaction membrane is located at the central position of the cylinder of the reaction chamber and vertically placed above the first reaction membrane. The testing process includes the following steps of:
(1) adding 2 ml lysis solution to 0.2 ml sample to complete sample lysis;
(2) adding 0.5 ml lysed sample from the sample inlet;
(3) completing the first rotation of the test device for nucleic acid;
(4) adding 0.06 ml eluant from the sample inlet;
(5) turning on a constant-temperature heating device for the isothermal amplification of nucleic acid with a temperature and time;
(6) completing the second rotation of the test device for nucleic acid; and
(7) reading a test result from the observation window of the test strip top cover.

### Example 4 Effects of immobilization or air-drying process on the test result

The test system for nucleic acid provided by Example 3 was applied for testing in this example; the object to be tested, nucleic acid, was a kind of feline herpes virus FHV-R; the sample to be tested was 2 copies/µL throat swab; the amplification conditions: 42°C and 12 min; a standard colorimetric card (as shown in Figure 17) was used for comparison during the process of reading the test result.

Primers in this examples are as follows:
Forward primer FHV-F: CTATGTTTCTTATGGATATGAGACTTTGTGAT
Reverse primer FHV-R: BIO-ATAGTTTTAACATTTCGACACCATTCATGTAG
Probe FHV-P2:
FAM-CGGTCGCCTTCATATTGGTTGGAACCTTTAAC(THF)AAGTATATGTTCCTAACAG-C3 spacer

### 1. Immobilizing process and effect verification

For the convenience of storage, production and operation, a liquid reagent (0.02 ml of the first reaction liquid and the second reaction liquid described in Example 3) was respectively added to a glass fiber disc having a diameter of 6 mm, and dried for 2 h at a temperature of 37°C and a humidity of 10%, thus preparing an immobilized reagent. Test results of the liquid reagent and immobilized reagent were compared synchronously. The test results are shown in Table 1.

**Table 1. Effect verification of the immobilizing process**

| Sampling template | 10⁻⁴ ng/µL FPV plasmid | 10⁻⁵ ng/µL FPV plasmid | 10⁻³ ng/µL MF plasmid | 10⁻⁴ ng/µL MF plasmid | Clinical specimen 127# | Clinical specimen 936# | Negative |
|---|---|---|---|---|---|---|---|
| Liquid reagent | G9 | G5 | G10 | G4 | G10 | G10 | 0 |
| Immobilized reagent | G10 | G6 | G10 | G5 | G10 | G9 | 0 |

As can be seen from Table 1, the immobilized reagent and the liquid reagent are used for testing; there are similar test results without obvious difference, and even the mean detection sensitivity of the immobilized reagent is slightly higher than that of the common liquid reagent.

### 2. Air-drying process and effect verification

Preparation method of the air-drying agent: a liquid reagent was added on a specific container without any other carrier, and dried for 4 h at a temperature of 37°C and a humidity of 10%, then taken out of the container to be flake. Test results of the liquid reagent, the immobilized reagent and the air-drying agent were compared synchronously. The test results are shown in Table 2.

**Table 2. Effect verification of the air-drying process**

| | Liquid reagent | Immobilized reagent | Air-drying agent |
|---|---|---|---|
| 10⁻⁵ ng/µL FPV plasmid | G7 | G7.5 | G7.5 |
| 10⁻⁵ ng/µL FPV plasmid | G6.5 | G7.5 | G7.5 |
| Negative | 0 | 0 | 0 |

As can be seen from Table 2, the air-drying agent, the immobilized reagent and the liquid reagent are used for testing; there is no obvious difference among the test results, and even the mean detection sensitivity of the immobilized reagent and the air-drying agent is slightly higher than that of the common liquid reagent.

To sum up, in this test system for nucleic acid, the way of adding a liquid reagent temporarily may be completely replaced by the way of placing an immobilized reagent or an air-drying agent in advance, thus making the testing process more convenient.

Example 5 Effects of the immobilizing way of PEG and other reagents in the nucleic acid amplification reagent on the test result

To achieve a good amplification effect, PEG need to be added in the reaction reagent; the PEG preferably has a molecular weight of 20000-40000. PEG used in the example has a molecular weight of 20000 (Order NO. A601790CAS: [25322-68-3], SangonBiotech). The immobilizing process was as follows: 0.02 ml of the liquid reagent was added to a glass fiber disc having a diameter of 6 mm and a thickness of 1 mm by a pipettor, and dried for 2 h at a temperature of 37°C and a humidity of 10%, thus preparing an immobilized reagent. In this example, the following 4 immobilizing ways were applied in the process of immobilizing the nucleic acid amplification reagent: 1, complete mixing immobilization (only one reaction membrane); 2, joint immobilization of a primer probe and an enzyme (first reaction membrane) and separate immobilization of PEG (second reaction membrane); 3, joint immobilization of a primer probe and PEG (first reaction membrane) and separate immobilization of an enzyme (second reaction membrane); 4, joint immobilization of an enzyme and PEG (first reaction membrane), and separate immobilization of a primer probe (second reaction membrane). The test system for nucleic acid provided by Example 3 was applied for testing in this example; the object to be tested, nucleic acid, was a kind of feline herpes virus FHV-R; the sample to be tested was 2 copies/µL throat swab; the amplification conditions: 42°C and 12 min; a standard colorimetric card (as shown in Figure 17) was used for comparison during the process of reading the test result. Effects of the 4 immobilizing ways on the test result were subjected to comparison. The results are shown in Table 3.

**Table 3 Effects of the immobilizing way of PEG and other reagents on the test result**

| Immobilizing way | Liquid reagent | Complete mixing immobilization | Primer probe + enzyme immobilization, separate immobilization of PEG | Primer probe + PEG immobilization, separate immobilization of enzyme | Enzyme + PEG immobilization, separate immobilization of primer probe |
|---|---|---|---|---|---|
| 10⁻⁴ ng/µL FPV plasmid | G9 | 0 | G9.5 | G3.5 | G3 |
| 10⁻⁵ ng/µL FPV plasmid | G7 | 0 | G7 | 0 | 0 |
| Negative | 0 | 0 | 0 | 0 | 0 |

As can be seen from Table 3, PEG and other reaction reagents need to be separated during the reagent immobilizing and drying. The reason may be as follows: PEG will be shrunk in the immobilizing process to wrap the enzyme or primer probe, and is difficult to restore activity after being redissolved in later period, which will cause the failure of the isothermal amplification of nucleic acid. Therefore, PEG need to be immobilized separately.

### Example 6 Selection for the nucleic acid adsorption membrane

The test system for nucleic acid provided by Example 3 was applied for testing in this example; the nucleic acid adsorption membrane in the test device for nucleic acid was respectively made of different membrane materials as shown in Table 4. the object to be tested, nucleic acid, was a kind of feline herpes virus FHV-R; the sample to be tested was 2 copies/µL throat swab; the amplification conditions: 42°C and 12 min; a standard colorimetric card (as shown in Figure 17) was used for comparison during the process of reading the test result. The membrane permeating time, water content, adsorption capacity and the like of different nucleic acid adsorption membranes were respective surveyed. The test results are shown in Table 4.

**Table 4 Selection for the nucleic acid adsorption membrane**

| Membrane material | Name | Pore diameter | Thickness | Membrane permeating time | Water content | Adsorption capacity |
|---|---|---|---|---|---|---|
| Silicon dioxide | GF/A | 1.6 µm | 260 µm | 13s | 4.0 µL | Ct20.10 |
| | GF/B | 1 µm | 675 µm | 15s | 8.5 µL | Ct19.72 |
| | GF/C | 1.2 µm | 260 µm | 6s | 2.9 µL | Ct20.42 |
| | GF/D | 2.7 µm | 675 µm | 10s | 5.3 µL | Ct21.35 |
| | GF/F | 0.7 µm | 420 µm | 39 s | 4.2 µL | Ct20.63 |
| Polysiloxane | Silica membrane | 1 µm | 350 µm | 10s | 4.0 µL | Ct20.00 |

As can be seen from Table 4, different membrane materials have greater differences in membrane permeating time. When the test device for nucleic acid provided by the present invention is used for testing, the sample directly permeates the nucleic acid adsorption membrane from the upper part; the shorter the membrane permeating time of the membrane material is, the faster the nucleic acid absorption rate in the same is. Therefore, a silicon dioxide membrane or a silica membrane of GF/C is adopted preferably. Because such two types of membranes have a small pore diameter, fast flow rate, low water holding capacity and good adsorbing effect, and thus are particularly suitable as the nucleic acid adsorption membrane. The silicon dioxide membrane of GF/C has a shorter membrane permeating time and the silica membrane has a better adsorption capacity (the lower the value of the adsorption capacity is, the better the adsorption capacity is).

### Example 7 Verification of the enrichment efficiency of the nucleic acid adsorption membrane

A silica membrane was selected as the nucleic acid adsorption membrane in this example to survey the test result of nucleic acid after being adsorbed by the nucleic acid adsorption membrane with the increase of the sample size of the membrane filtering, thus judging the enrichment efficiency of the nucleic acid adsorption membrane. The object to be tested, nucleic acid, was a kind of feline herpes virus FHV-R; the sample to be tested was 2 copies/µL throat swab; the amplification conditions: 42°C and 12 min; test method: PCR fluorescence or product test strip was respectively applied for testing. Results are shown in Table 5.

**Table 5 Verification of the enrichment efficiency of the nucleic acid adsorption membrane**

| Membrane filtering sample Volume | Elution volume | PCR fluorescence testing | | | Product test strip testing | | |
|---|---|---|---|---|---|---|---|
| 10 µL | 100 µL | UN | 32.8 | UN | G7 | G7.5 | G7 |
| 50 µL | 100 µL | UN | 31.83 | UN | G8 | G7 | G7 |
| 250 µL | 100 µL | 27.16 | 28.36 | UN | G7.5 | G8 | G8 |
| 500 µL | 100 µL | 26.25 | 28.39 | 27.29 | G8.5 | G8 | G9 |
| 1000 µL | 100 µL | 27.16 | 27.47 | 26.74 | G8 | G9 | G8.5 |

As can be seen from Table 5, with the increase of the volume of the membrane filtering sample, either the fluorescent Ct value or the color depth of the colloidal gold test strip is improved more significantly, indicating that the silica membrane has better enrichment effects for nucleic acid.

### Example 8 Effects of different lysis solution on the test result

The test system for nucleic acid provided by Example 3 was applied for testing in this example; the lysis solution was respectively selected from the table below, thus surveying the effects of different lysis solution on the test result of nucleic acid; the object to be tested, nucleic acid, was a kind of feline herpes virus FHV-R; the sample to be tested was 2 copies/µL throat swab; the amplification conditions: 42°C and 12 min. Test results are shown in Table 6.

**Table 6 Effects of different lysis solution on the test result**

| No. | Formula of the lysis solution | Test result |
|---|---|---|
| 1 | Tris + EDTA + guanidinium isothiocyanate | G3 |
| 2 | Tris + guanidinium isothiocyanate + Tween 20 + NaCl | G4 |
| 3 | Tris + guanidinium isothiocyanate + Tween 20 | G5 |
| 4 | Tris + EDTA + guanidinium isothiocyanate+ Tween 20 | G6 |
| 5 | Tris + EDTA + guanidinium isothiocyanate + Triton 100 | G8 |

As can be seen from Table 6, different lysis solution has significant influences on the test result. The reason may be as follows: some lysis solution may cause adverse effect on the isothermal amplification after flowing into the nucleic acid amplification chamber, but the lysis solution provided by the present invention may not cause adverse effect on the nucleic acid amplification. Therefore, after the nucleic acid in the sample is absorbed by the nucleic acid adsorption membrane, it is unnecessary to add a cleaning agent for washing and the target nucleic acid may be eluted by an eluant for isothermal amplification; therefore, the lysis solution provided herein is particularly suitable for the test device for nucleic acid provided in the present invention.

### Example 9 Effects of different eluant on the test result

The test system for nucleic acid provided by Example 3 was applied for testing in this example; the eluant was respectively selected from the table below, thus surveying the effects of different eluant on the test result of nucleic acid; the object to be tested, nucleic acid, was a kind of feline herpes virus FHV-R; the sample to be tested was 2 copies/µL throat swab; the amplification conditions: 42°C and 12 min. Test results are shown in Table 7.

**Table 7 Effects of different eluant on the test result**

| No. | Formula of the eluant | Test result |
|---|---|---|
| 1 | Tris | Not detected |
| 2 | EDTA | Not detected |
| 3 | Magnesium acetate | Not detected |
| 4 | Tris + EDTA | G5 |
| 5 | Tris + EDTA + magnesium acetate | G8 |

As can be seen from Table 7, different eluant has significant influences on the test result. The reason may be as follows: after being eluted by the eluant, the nucleic acid in the nucleic acid adsorption membrane is required to flow into the reaction chamber to be mixed with the immobilized reagent for nucleic acid amplification for isothermal amplification. Moreover, an eluant which will not cause adverse effect on the isothermal amplification of nucleic acid and even may effectively promote the isothermal amplification of nucleic acid needs to be selected and used in the test device for nucleic acid. Therefore, the eluant in the group 5 is preferably used.

### Example 10 Effects of the width and number of different drainage channels on the detection sensitivity of nucleic acid

The test device for nucleic acid provided by Example 1 was applied and combined with a nucleic acid detection reagent for testing in this example. The width and number of the drainage channels forming the drainage groove are respectively as shown in Table 8 to detect the detection sensitivity of nucleic acid under different configuration conditions. The object to be tested, nucleic acid, was a kind of feline herpes virus FHV-R; the sample to be tested was 2 copies/µL throat swab; the amplification conditions: 42°C and 12 min. Test results are shown in Table 8.

### Example 8 Effects of the width and number of different drainage channels on the detection sensitivity of nucleic acid

| No. | Width of the drainage channel (mm) | Number of the drainage channel (pcs.) | Detected frequency/Detection times |
|---|---|---|---|
| 1 | 0.3 | 15 | 19/20 |
| 2 | | 14 | 19/20 |
| 3 | 0.5 | 14 | 19/20 |
| 4 | | 13 | 20/20 |
| 5 | 0.7 | 13 | 17/20 |
| 6 | | 12 | 17/20 |
| 7 | 0.9 | 12 | 16/20 |
| 8 | | 11 | 15/20 |

As can be seen from Table 8, the detection sensitivity of nucleic acid is improved significantly with the decrease of the width of the drainage channel, but when the width of the drainage channel narrows to 0.3 mm, the improvement amplitude of the sensitivity slows down obviously. As can be seen, it is very limited for the improvement of the sensitivity to further narrow the width of the drainage channel, meanwhile, the smaller the width of the drainage channel is, the more difficult the manufacturing process is, and the higher the cost is. Therefore, the width of the drainage channel is preferably 0.3-0.7 mm, and most preferably 0.5 mm.

Meanwhile, the number of the drainage channel also causes a certain impact on the detection sensitivity of nucleic acid; when the width of the drainage channel is not less than 0.5 mm, the increase of the drainage channel may further improve the detection sensitivity of nucleic acid. However, when the width of the drainage channel is less than 0.5 mm, the increase of the drainage channel has no obvious effect on the improvement of the detection sensitivity of nucleic acid. Therefore, in view of the difficulty level of manufacture and costs, the width of the drainage channel is preferably 0.5 mm, and the number of the drainage channel is 13.

### Example 11 Effects of the height of the sampling channel on the test result

The test system for nucleic acid provided by Example 3 was applied in this example; the height of the sampling channel in the test device for nucleic acid was respectively selected from the table below, thus surveying the effects of the height of the sampling channel on the test result. The object to be tested, nucleic acid, was a kind of feline herpes virus FHV-R; the sample to be tested was 2 copies/µL throat swab; the amplification conditions: 42°C and 12 min. Test results are shown in Table 9.

**Table 9 Effects of the height of the sampling channel on the test result**

| No. | Height of the sampling channel (mm) | Detected frequency/Detection times |
|---|---|---|
| 1 | 11 | 17/20 |
| 2 | 15 | 19/20 |
| 3 | 19 | 20/20 |
| 4 | 23 | 20/20 |
| 5 | 27 | 20/20 |

As can be seen from Figure 9, different height of the sampling channel will directly affect the test result of nucleic acid in the sample. The reason may be as follows: different height of the sampling channel will affect the adsorption effect of the nucleic acid adsorption membrane on the sample; the sample enters from the sample inlet and is capable of impacting the nucleic acid adsorption membrane with greater force after being accelerated by a certain height of sampling channel, which promotes the nucleic acid adsorption membrane to adsorb the nucleic acid in the sample more fully, thereby improving the detection sensitivity of nucleic acid. Therefore, the sampling channel having a height of 19 mm is used preferably.

Example 12 Effects of the arrangement way of the first reaction membrane and the second reaction membrane on the test result

The test system for nucleic acid provided by Example 3 was applied in this example; a way of placing the first reaction membrane and the second reaction membrane in advance was applied for the nucleic acid amplification reagent; there are three ways of arrangement to the first reaction membrane and the second reaction membrane, respectively as follows: 1, the first reaction membrane and the second reaction membrane are placed horizontally; 2, the second reaction membrane is vertically placed above the first reaction membrane; 3, the second reaction membrane and the first reaction membrane are placed vertically. The detection sensitivity of nucleic acid was detected under different ways of arrangement of the reaction membrane; the object to be tested, nucleic acid, was a kind of feline herpes virus FHV-R; the sample to be tested was 2 copies/µL throat swab; the amplification conditions: 42°C and 12 min. Test results are shown in Table 10.

**Table 10 Effects of the different arrangement ways of the reaction membrane on the test result**

| No. | Arrangement way | Detected frequency/Detection times |
|---|---|---|
| 1 | The first reaction membrane and the second reaction membrane are arranged horizontally | 17/20 |
| 2 | Further, the first reaction membrane is arranged horizontally, and the second reaction membrane is vertically arranged above the first reaction membrane | 20/20 |
| 3 | The first reaction membrane and the second reaction membrane are arranged vertically | 16/20 |

As can be seen from Table 10, the second arrangement way was applied, namely, the first reaction membrane is arranged horizontally, and the second reaction membrane is vertically arranged above the first reaction membrane. Such a configuration way may obviously improve the detection sensitivity of nucleic acid. The reason may be as follows: the second reaction membrane may also play a role of drainage to a certain extent, meanwhile, the second reaction membrane may further improve the mixing effect of the reagents in the first reaction membrane and the second reaction membrane. Therefore, the arrangement way that the second reaction membrane is vertically arranged above the first reaction membrane is applied preferably.

The test system for nucleic acid provided by this example may be used in on-site test, clinics, pet stores and the like, and may be used for nucleic acid testing of pathogens, such as pathogens of pets, pathogens of large livestock, plant pathogens or food pathogens.

These below embodiments are also as the part of the present invention.

A test system for nucleic acid, comprising a test device for nucleic acid and a reagent, wherein the reagent comprises a lysis solution, an eluant and a nucleic acid amplification reagent; the nucleic acid amplification reagent is a dry immobilized reagent, and comprises a first immobilized reagent and a second immobilized reagent; the first immobilized reagent contains an enzyme, and the second immobilized reagent contains PEG.

In some embodiments, the first immobilized reagent further contains a primer probe, a single-strand binding protein, a protein cofactor, and DNTP.

In some embodiments, the test device for nucleic acid comprises a sample treatment chamber, a sample reaction chamber and a test chamber which are disposed from top to bottom successively; the sample treatment chamber is used for adsorbing nucleic acid in the sample; the sample reaction chamber is used for completing the nucleic acid amplification; the test chamber is used for detecting nucleic acid in the amplified product; the sample reaction chamber comprises a reaction chamber used for nucleic acid amplification; the nucleic acid amplification reagent is disposed in the reaction chamber of the sample reaction chamber in advance.

In some embodiments, the nucleic acid amplification reagent is an immobilization membrane or a drying agent.

In some embodiments, when the nucleic acid amplification reagent is an immobilization membrane, the immobilization membrane comprises a first reaction membrane and a second reaction membrane; the first reaction membrane contains a first immobilized reagent, and the second reaction membrane contains a second immobilized reagent; when the nucleic acid amplification reagent is a drying agent, the drying agent comprises a first drying agent and a second drying agent; the first drying agent contains a first immobilized reagent, and the second drying agent contains a second immobilized reagent; and when the nucleic acid amplification reagent is a drying agent, a division bar need to be disposed at the bottom of the reaction chamber; and the first drying agent is spaced from the second drying agent by the division bar.

In some embodiments, the lysis solution comprises Tris, ethylene diamine tetraacetic acid (EDTA), guanidinium isothiocyanate and Triton 100.

In some embodiments, the sample treatment chamber comprises a nucleic acid adsorption membrane, and the nucleic acid adsorption membrane is a silicon dioxide GF/C membrane or a silica membrane.

In some embodiments, wherein the eluant comprises magnesium acetate, Tris and ethylene diamine tetraacetic acid (EDTA).

In some embodiments, wherein the sample treatment chamber is provided with a sample inlet without a cover; the lysis solution or the eluant is placed into a reagent bottle; and the reagent bottleneck is capable of being sealed with the sample inlet.

Any content not covered in the present invention is common general knowledge. Even though the present invention has been disclosed as above, the present invention is not limited thereto. Any person skilled in art can make various changes and modifications within the spirit and scope of the present invention. Therefore, the protection scope of the present invention shall be subjected to the scope defined in the claims.

## Claims

1. A test device, comprising:
a chamber used for treating a sample;
a chamber used for sample reaction, wherein a reaction product of the sample is obtained in the chamber; and
a test chamber used for detecting the reaction product;
wherein the sample treatment chamber has a first position, a second position and a third position;
wherein the sample reaction chamber has a first position and a second position; and when the sample treatment chamber and the sample reaction chamber are located in the first position, the sample treatment chamber, the sample reaction chamber and the test chamber are not in fluidic communication with each other.

2. The test device according to claim 1, wherein when the sample treatment chamber is located in the second position, the sample treatment chamber is in fluidic communication with the sample reaction chamber, and the sample reaction chamber is not in fluidic communication with the test chamber.

3. The test device according to claim 2, wherein when the sample treatment chamber is located in the third position, the sample reaction chamber is in fluidic communication with the test chamber.

4. The test device according to one of claims 1-2, wherein when the sample treatment chamber moves to the second position from the first position, the sample reaction chamber is located in the first position; when the sample treatment chamber moves to the third position from the second position, the sample reaction chamber is located in the second position, or the sample reaction chamber moves to the second position from the first position.

5. The test device according to one of claims 1-4, wherein when the sample reaction chamber is located in the second position, the sample reaction chamber is in fluidic communication with the test chamber.

6. The test device according to one of claims 1-5, wherein the sample reaction chamber moves to the second position from the first position while the sample treatment chamber moves to the third position from the second position; or the sample treatment chamber and the sample reaction chamber move simultaneously, thus driving the sample treatment chamber to move to the third position from the second position and driving the sample reaction chamber to move to the second position from the first position.

7. The test device according to one of claims 1-6, wherein the sample treatment chamber, the sample reaction chamber and the test chamber are disposed from top to bottom successively.

8. The test device according to one of claims 1-7, wherein transformation of the sample treatment chamber and the sample reaction chamber is achieved by rotation in different positions.

9. The test device according to claim 8, wherein the rotation comprises two rotations, wherein the first rotation causes the sample treatment chamber to move to the second position from the first position; and the sample reaction chamber and the test chamber keep still; at the end of the first rotation, the sample treatment chamber is in fluidic communication with the sample reaction chamber, and the sample reaction chamber is not in fluidic communication with the test chamber.

10. The test device according to claim 9, wherein the second rotation causes the sample treatment chamber to move to the third position from the second position; and meanwhile, the sample reaction chamber moves to the second position from the first position; at the end of the second rotation, the sample treatment chamber, the sample reaction chamber and the test chamber are in fluidic communication with each other.

11. The test device according to one of claims 1-10, wherein the sample treatment chamber is used for extracting nucleic acid substances from the sample and wherein the sample reaction chamber is used for amplifying the nucleic acid substances, thus producing an amplified product.

12. The test device according to claim 11, wherein the test chamber is used for detecting the number of the amplified product or detecting the presence or absence of the amplified product.

13. The test device according to claim 13, wherein the sample reaction chamber comprises a reagent for nucleic acid amplification and the reagent exists in a dry state.

14. The test device according to claim 13, wherein the sample reaction chamber is provided with a reaction chamber and a filter paper storage slot; the filter paper storage slot is provided with filter paper used for adsorbing excessive samples; the reaction chamber is provided with a nucleic acid amplification membrane or fixed with a nucleic acid amplification drying agent; the test chamber is provided with a sampling hole, and the reaction product flows into the transverse flow test strip from the sampling hole to start testing.

15. The test device according to claim 14, wherein when the sample treatment chamber, the sample reaction chamber and the test chamber are not communicated, the sample inlet of the sample treatment chamber is vertically communicated with the filter paper storage slot; when the sample treatment chamber is communicated with the sample reaction chamber, the sample inlet of the sample treatment chamber is vertically communicated with the reaction chamber of the sample reaction chamber; when the sample treatment chamber, the sample reaction chamber and the test chamber are communicated, the sample inlet of the sample treatment chamber, the reaction chamber of the sample reaction chamber and the sampling hole of the test chamber are communicated vertically.
